(19) Europäisches Patentamt
European Patent Office
Office européen des brevets

(11) EP 2 002 836 B1

(12) **EUROPEAN PATENT SPECIFICATION**

(45) Date of publication and mention of the grant of the patent:
**20.10.2010 Bulletin 2010/42**

(51) Int Cl.:
***A61K 31/437*** *(2006.01)* ***C07D 471/06*** *(2006.01)*
***C07D 471/16*** *(2006.01)* ***A61P 35/00*** *(2006.01)*

(21) Application number: **08156203.5**

(22) Date of filing: **14.05.2008**

(54) **Cyclocondensed azaindoles active as kinase inhibitors**

Zyklokondensierte Azaindole, die als Kinase-Hemmer wirken

Azaindoles cyclocondensés actifs en tant qu'inhibiteurs de la kinase

(84) Designated Contracting States:
**DE FR GB IT**

(30) Priority: **31.05.2007 EP 07109338**

(43) Date of publication of application:
**17.12.2008 Bulletin 2008/51**

(73) Proprietor: **Nerviano Medical Sciences S.r.l.**
**20014 Nerviano (MI) (IT)**

(72) Inventors:
- **Casuscelli, Francesco**
  **20015, Parabiago (MI) (IT)**
- **Casale, Elena**
  **21019, Somma Lombardo (VA) (IT)**
- **Faiardi, Daniela**
  **27100, Pavia (IT)**
- **Mongelli, Nicola**
  **20137, Milan (IT)**
- **Piutti, Claudia**
  **20014, Nerviano (MI) (IT)**
- **Traquandi, Gabriella**
  **20151, Milan (IT)**

(56) References cited:
**WO-A-03/008414** **WO-A-2005/063747**
**WO-A-2006/068796** **WO-A-2006/118749**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 002 836 B1

## Description

## BACKGROUND OF THE INVENTION

### Field of the invention

The present invention relates to certain substituted cyclocondensed azaindole compounds, which modulate the activity of protein kinases. The compounds of this invention are therefore useful in treating diseases caused by disregulated protein kinase activity. The present invention also provides methods for preparing these compounds, pharmaceutical compositions comprising these compounds, and methods of treating diseases utilizing pharmaceutical compositions comprising these compounds.

### Discussion of the background

The malfunctioning of protein kinases (PKs) is the hallmark of numerous diseases. A large share of the oncogenes and proto-oncogenes involved in human cancers code for PKs. The enhanced activities of PKs are also implicated in many non-malignant diseases, such as benign prostate hyperplasia, familial adenomatosis, polyposis, neuro-fibromatosis, psoriasis, vascular smooth cell proliferation associated with atherosclerosis, pulmonary fibrosis, arthritis glomerulonephritis and post-surgical stenosis and restenosis.

PKs are also implicated in inflammatory conditions and in the multiplication of viruses and parasites. PKs may also play a major role in the pathogenesis and development of neurodegenerative disorders.

For a general reference to PKs malfunctioning or disregulation see, for instance, Current Opinion in Chemical Biology 1999, 3, 459 - 465.

AKT (also known as protein kinase B (PKB) or Rac-PK-beta), and its gene family products, has been identified as a serine/threonine protein kinase (Proc. Natl. Acad. Sci. 2001, 98, 10983-10985; J. Cell. Sci. 2001, 114, 2903-2910; Circ. Res. 2000, 86, 15-23). Three isoforms of PKB are currently known, PKBα (AKT1), PKBβ (AKT2), and PKBγ (AKT3) (Proc. Natl. Acad. Sci. 1992, 89, 9267-9271; J. Biol. Chem. 1999, 274, 9133-9136). PKB mediates many effects of IGF-1 and other growth factors on tumor growth and inhibition of apoptosis (Cell. Signal. 2002, 14, 381-395). PKB plays an important role in cell proliferation, apoptosis and response to insulin. For these reasons, modulation of PKBs is of interest in the treatment of tumorigenesis, abnormal cell proliferation and diabetes. The molecular structure of the PKBs comprises a regulatory site near the carboxy terminus of the polypeptide, a catalytic domain with an activation loop having a threonine, and an amino-terminal pleckstrin homology domain. The pleckstrin homology domain permits anchorage of the enzyme to the cell membrane through interaction with phospholipids, which triggers the activation of the PKBs. The role of pleckstrin homology domain requires phosphorylation of phosphatidylinositol at the D-3 position via phosphatidylinositol 3-kinase PI3K, an SH2 domain protein that associates with activated receptor tyrosyne kinases, particularly IGF-1R. In particular, phosphoinositol-3-kinase, when activated by receptor tyrosine kinase, catalyzes the synthesis of phosphoinositol-3,4-diphosphate and phosatidylinositol-3,4,5-triphosphate. The pleckstrin homology domain binds 3-phosphoinositides, which are synthesized by PI3K upon stimulation by growth factors such as platelet derived growth factor (PDGF), nerve growth factor (NGF) and insulin-like growth factor (IGF-1) (Mol. Cell. Biol. 1997, 17, 1595-1606; Science 1997, 275, 628-630; Genev. Dev. 1999, 13, 2905-2927). Lipid binding to the pleckstrin homology domain promotes translocation of PKB to the plasma membrane. Further activation of PKB occurs by phosphorylation by another protein kinase, PDK1 at Thr308, Thr309 and Thr305 for the PKB isoforms 1, 2 and 3, respectively. A third step of activation is catalyzed by a kinase that phosphorylates Ser473, Ser474 or Ser472 in the C-terminal tails ofPKB/AKT-1, -2 and -3 respectively. The Ser473 kinase activity has been identified to be associated with plasma membrane and is not due to PKB and PDK1 kinase activity (Current Biology 2002, 12, 1251-1255; J. Biol. Chem. 2003, 278, 21615-21622). The process produces the fully activated form of PKB.

Activation of PKB can also occur by inhibiting the D-3 phosphoinositide specific phosphatase, PTEN, which is a membrane associated FYVE finger phosphatase commonly inactivated in many cancers, including prostate cancer (Eur. J. Biochem. 1999, 263, 605-611; Cancer Res. 1997, 57, 2124-2129).

The catalytic domain of PKB is responsible for the phosphorylation of serine or threonine in the target protein.

Once activated, PKB mediates several cellular functions, including proliferation, cell growth, and promotion of survival.

The antiapoptotic function of PKB is reported to be mediated by its ability to phosphorylate apoptosis regulatory molecules including BAD, caspase 9, IKK-, and the forkhead transcriptional factor FKHRL1. PKB signal is also implicated in the physiological regulation of organ size (Nat. Cell. Biol. 1999, 1, 500-506), glucose homeostasis (J. Biol. Chem. 1999, 274, 1865-1868), vasomotor tone (J. Clin. Invest. 1999, 106, 493-499), and angiogenesis (Nat. Med. 2000, 6, 1004-1010).

Manifestations of altered PKB regulation appear in both injury and disease, the most important role being in

cancer. PKB kinase activity is constitutively activated in tumors with PTEN mutation, PI3-kinase mutation and overexpression, and receptor tyrosin kinase overexpression. PKB is also a mediator of normal cell functions in response to growth factor signalling. Expression of the AKT gene was found to be amplified in 15 % of human ovarian carcinoma cases (Proc. Natl. Acad. Sci. 1992, 89, 9267-9271). AKT is also overexpressed in 12 % of pancreatic cancers (Proc. Natl. Acad. Sci 1996, 93, 3636-3641). In particular, AKT-2 is overexpressed in 12 % of ovarian carcinomas and in 50 % of indifferentiated tumors, suggesting that PKB may be associated with tumor aggressiveness (Int. J. Cancer 1995, 64, 280-285). PKB is also a mediator of normal cell functions (Nature 1999, 401, 33-34; Oncogene 2000, 19, 2324-2330; Neurosci. 2000, 20, 2875-2886).

Elucidation of the role of PKB in the increase of growth and inhibition of apoptosis is complicated by the many protein substrates of PKB, including BAD, Forkhead (FOXO family), GSK3, Tuberin (TSC2), p27 Kip1, p70S6k, protein kinase C-, forkhead in rhabdomyosarcoma, Raf, cAMP-response element-binding protein, SGK-1, m-TOR, and the androgen receptor (Proc. Natl. Acad. 2001, 98, 7200-7205; Nature 2001, 411, 355-365; Nat. Rev. Cancer 2002, 2, 489-501).

The various PKBs vary in their abundance in different mammalian cell types. For example, PKB β are especially abundant in highly insulin-responsive tissues, including brown fat.

Modulation of PKB by small molecules can be achieved by identifying compounds that bind to and activate or inhibit one or more PKBs.

It is an object of the invention to provide compounds which are useful, in therapy, as agents against a host of diseases caused by and/or associated to a disregulated protein kinase activity and, more particularly, AKT known also as protein kinase B activity.

## SUMMARY OF THE INVENTION

It has been found that compounds of formula (I) described below, are kinase inhibitors and are thus useful in therapy as antitumor agents. Accordingly, a first object of the present invention is to provide a substituted cyclocondensed azaindole compound represented by formula (I),

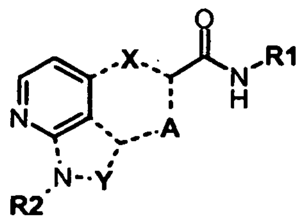

(I)

wherein

**R1** is hydrogen or an optionally substituted group selected from straight or branched $C_1$-$C_6$ alkyl, $C_3$-$C_6$ cycloalkyl, heterocycloalkyl, aryl, arylalkyl, heteroaryl and a group of formula (II):

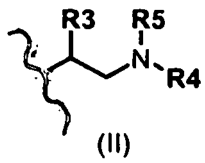

(II)

**R2** is hydrogen or an optionally substituted group selected from straight or branched $C_1$-$C_6$ alkyl, aryalkyl and a group -CO**R7**;

**R3** is an optionally substituted group selected from straight or branched $C_1$-$C_6$ alkyl, $C_3$-$C_6$ cycloalkyl, heterocycloalkyl, aryl, arylalkyl and heteroaryl;

**R4** and **R5** are hydrogen, or taken together with the nitrogen atom to which they are bonded, may form an optionally substituted heterocycloalkyl group;

**R7** is an optionally substituted group selected from straight or branched $C_1$-$C_6$ alkyl, $C_3$-$C_6$ cycloalkyl, heterocy-

cloalkyl, aryl, arylalkyl and heteroaryl;
**X** is -N**(R6)**-$CH_2$-, **A** is -$CH_2$-, **Y** is -CH= and **R6** is hydrogen or a group -CO**R7,** or
**X** is -N**(R6)**$CH_2$-, **A** is -CH=, **Y** is -CH= and **R6** is hydrogen or a group -CO**R7,** or
**X** is -$CH_2$-$CH_2$, **A** is -CH= and **Y** is -CH=, or
**X** is -CH=, **A** is -CH= and **Y** is -$CH_2$-, and

the dotted lines may represent a single, double or aromatic bond;or isomers, tautomers, carriers, complexes, metabolites, prodrugs, solvates, hydrates or pharmaceutically acceptable salts thereof.

The present invention also provides a method for treating diseases caused by and/or associated with dysregulated protein kinase activity, particularly one or more isoforms of the serin/threonine kinase AKT, PLK family, protein kinase C in different isoforms, Met, PAK-4, PAK-5, ZC-1, STLK-2, DDR-2, Aurora 1, Aurora 2, Bub-1, Chk1, Chk2, HER2, raf1, MEK1, EGF-R, PDGF-R, FGF-R, IGF-R, ALK , PI3K, weel kinase, Src, Abl, MAPK, ILK, MK-2, IKK-2, Cdc7, Nek, Cdk/cyclin kinase family, CK2, GSK3, SULU, PDK1, RET, KIT, LCK, TRKA, PKA$\alpha$, SGK1, SULU1, ERK2, VEGFR3, PDGFR, more particulary one or more isoforms of the serin/threonine kinase AKT, which comprises administering to a mammal in need thereof an effective amount of a substituted cyclocondensed azaindole compound represented by formula (I) as defined above.

A preferred method of the present invention is to treat a disease caused by and/or associated with dysregulated protein kinase activity selected from the group consisting of cancer and cell proliferative disorders.

Another preferred method of the present invention, is to treat a variety of cancers including, but not limited to: carcinoma such as bladder, breast, colon, kidney, liver, lung, including small cell lung cancer, esophagus, gall-bladder, ovary, pancreas, stomach, cervix, thyroid, prostate, and skin, including squamous cell carcinoma; hematopoietic tumors of lymphoid lineage, including leukemia, acute lymphocitic leukemia, acute lymphoblastic leukemia, B-cell lymphoma, T-cell-lymphoma, Hodgkin's lymphoma, non-Hodgkin's lymphoma, hairy cell lymphoma and Burkitt's lymphoma; hematopoietic tumors of myeloid lineage, including acute and chronic myelogenous leukemias, myelodysplastic syndrome and promyelocytic leukemia; tumors of mesenchymal origin, including fibrosarcoma and rhabdomyosarcoma; tumors of the central and peripheral nervous system, including astrocytoma, neuroblastoma, glioma and schwannomas; other tumors, including melanoma, seminoma, teratocarcinoma, osteosarcoma, xeroderma pigmentosum, keratoxanthoma, thyroid follicular cancer and Kaposi's sarcoma. Another preferred method of the present invention, is to treat cell proliferative disorders such as, but not restricted to, benign prostate hyperplasia, familial adenomatosis polyposis, neurofibromatosis, psoriasis, vascular smooth cell proliferation associated with atherosclerosis, pulmonary fibrosis, arthritis, glomerulonephritis, post-surgical stenosis and restenosis and Alzheimer's disease.

In addition, the method of the present invention also provides tumor angiogenesis and metastasis inhibition.

In a further preferred embodiment, the method of the present invention further comprises subjecting the mammal in need thereof to a radiation therapy or chemotherapy regimen in combination with at least one cytostatic or cytotoxic agent. Moreover the invention provides a method for inhibiting the activity of one or more isoforms of the serin/threonine kinase AKT protein which comprises contacting the said protein with an effective amount of a compound of formula (I).

The present invention also provides a pharmaceutical composition comprising one or more compounds of formula (I) or a pharmaceutically acceptable salt thereof and a pharmaceutically acceptable excipient, carrier or diluent.

The present invention further provides a pharmaceutical composition comprising a compound of formula (I) in combination with one or more chemotherapeutic agents or radiotherapy. Such agents can include, but are not limited to, cytostatic or cytotoxic agents, antibiotic-type agents, alkylating agents, antimetabolite agents, hormonal agents, antihormonal agents such as antiestrogens, antiandrogens and aromatase inhibitors, immunological agents, interferon-type agents, cyclooxygenase inhibitors (e.g. COX-2 inhibitors), matrixmetalloprotease inhibitors, telomerase inhibitors, kinase inhibitors, anti-growth factor receptor agents, anti-HER agents, anti-EGFR agents, anti-angiogenesis agents (e.g. angiogenesis inhibitors), farnesyl transferase inhibitors, ras-raf signal transduction pathway inhibitors, cell cycle inhibitors, other cdks inhibitors, tubulin binding agents, topoisomerase I inhibitors, topoisomerase II inhibitors, agents that target microtubules, platin-based agents, DNA damaging or intercalating agents, inhibitors of kinesins, therapeutic monoclonal antibodies, inhibitors of mTOR, histone deacetylase inhibitors, and inhibitors of hypoxic response.

Additionally, the invention provides a product or kit comprising a compound of formula (I) or a pharmaceutically acceptable salt thereof, as defined above, or pharmaceutical compositions thereof and one or more chemotherapeutic agents, as a combined preparation for simultaneous, separate or sequential use in anticancer therapy.

In yet another aspect the invention provides a compound of formula (I) or a pharmaceutically acceptable salt thereof, as defined above, for use as a medicament. Moreover the invention provides the use of a compound of formula (I) or a pharmaceutically acceptable salt thereof, as defined above, in the manufacture of a medicament with antitumor activity.

Finally, the invention provides a compound of formula (I) or a pharmaceutically acceptable salt thereof, as defined above, for use in a method of treating cancer.

## DETAILED DESCRIPTION OF THE INVENTION

Indole- and azaindole- containing amide as antagonists TGF-β are described in WO2003037862 in the name of Nippon Shinyaku Co., Japan.

Azaindolacrylamide derivatives as kinase inhibitors are disclosed in WO200198299 in the name of Pharmacia.

Unless otherwise specified, when referring to the compounds of formula (I) per se as well as to any pharmaceutical composition thereof or to any therapeutic treatment comprising them, the present invention includes all of the isomers, tautomers, carriers, complexes, metabolites, prodrugs, solvates, hydrates and pharmaceutically acceptable salts of the compounds of this invention.

If a chiral center or another form of an isomeric center is present in a compound of the present invention, all forms of such isomer or isomers, including enantiomers and diastereomers, are intended to be covered herein. Compounds containing a chiral center may be used as a racemic mixture, an enantiomerically enriched mixture, or the racemic mixture may be separated using well-known techniques and an individual enantiomer may be used alone. In cases in which compounds have unsaturated carbon-carbon double bonds, both the cis (Z) and trans (E) isomers are within the scope of this invention.

In cases wherein compounds may exist in tautomeric forms, such as keto-enol tautomers, each tautomeric form is contemplated as being included within this invention whether existing in equilibrium or predominantly in one form.

A metabolite of a compound of formula (I) is any compound into which this same compound of formula (I) is converted *in vivo,* for instance upon administration to a mammal in need thereof. Typically, without however representing a limiting example, upon administration of a compound of formula (I), this same derivative may be converted into a variety of compounds, for instance including more soluble derivatives like hydroxylated derivatives, which are easily excreted. Hence, depending upon the metabolic pathway thus occurring, any of these hydroxylated derivatives may be regarded as a metabolite of the compounds of formula (I).

Prodrugs are any covalently bonded compounds, which release in vivo the active parent drug according to formula (I).

The general terms as used herein, unless otherwise specified, have the meaning reported below.

The term "straight or branched $C_1$-$C_6$ alkyl" refers to a saturated aliphatic hydrocarbon radical, including straight chain and branched chain groups of from 1 to 6 carbon atoms, e.g. methyl, ethyl, propyl, 2-propyl, n-butyl, iso-butyl, tert-butyl, pentyl and the like. The alkyl group may be substituted or unsubstituted.

The term "$C_3$-$C_6$ cycloalkyl" refers to a 3- to 6-membered all-carbon monocyclic ring, which may contain one or more double bonds but does not have a completely conjugated π-electron system. Examples of cycloalkyl groups, without limitation, are cyclopropanyl, cyclobutanyl, cyclopentanyl, cyclopentenyl, cyclohexanyl, cyclohexenyl and cyclohexadienyl. A cycloalkyl group may be substituted or unsubstituted.

The term "heterocycloalkyl" refers to a 3- to 7-membered, saturated or partially unsaturated carbocyclic ring where one or more carbon atoms are replaced by heteroatoms such as nitrogen, oxygen and sulfur. Not limiting examples of heterocycloalkyl groups are, for instance, oxiranyl, aziridinyl, oxetanyl, azetidinyl, tetrahydrofuranyl, dihydrofuranyl, tetrahydrothiophenyl, dihydrothiophenyl, pyrrolidinyl, dihydropyrrolyl, pyranyl, dihydropyranyl, tetrahydropyranyl, tetrahydrothiopyranyl, piperidinyl, pyrazolinyl, isoxazolidinyl, isoxazolinyl, thiazolidinyl, thiazolinyl, isothiazolinyl, dioxanyl, piperazinyl, morpholinyl, thiomorpholinyl, examethyleneiminyl, homopiperazinyl and the like. A heterocycloalkyl group may be substituted or unsubstituted.

The term "aryl" refers to any aromatic mono-, bi- or poly-carbocyclic hydrocarbon with from 1 to 4 ring systems, either fused or linked to each other through a single bond, for instance including phenyl, α- or β-naphthyl or biphenyl groups. An aryl group may be substituted or unsubstituted.

The term "heteroaryl" refers to any aromatic heterocyclic ring which may comprise an optionally benzocondensed 5 or 6 membered heterocycle with from 1 to 3 heteroatoms selected among N, O or S.

Non limiting examples of heteroaryl groups according to the invention may thus include, for instance, pyridyl, pyrazinyl, pyrimidinyl, pyridazinyl, indolyl, imidazolyl, thiazolyl, isothiazolyl, pyrrolyl, phenyl-pyrrolyl, furyl, phenyl-furyl, oxazolyl, isoxazolyl, pyrazolyl, thienyl, benzothienyl, isoindolinyl, benzoimidazolyl, quinolinyl, isoquinolinyl, 1,2,3-triazolyl, 1-phenyl-1,2,3-triazolyl, 2,3-dihydroindolyl, 2,3-dihydrobenzofuranyl, 2,3-dihydrobenzothiophenyl; benzopyranyl, 2,3-dihydrobenzoxazinyl, 2,3-dihydroquinoxalinyl and the like. An heteroaryl group may be substituted or unsubstituted.

According to the present invention and unless otherwise provided, any of the above R1, R2, R3, R4, R5, R6 and R7 group may be optionally substituted, in any of their free positions, by one or more groups, for instance 1 to 3 groups, preferably one or two, independently selected from the groups consisting of halogen, cyano, amino, nitro, hydroxy, alkoxy, trifluoromethyl. In their turn, whenever appropriate, each of the above substituent may be further substituted by one or more of the aforementioned groups.

In this respect the term "halogen" indicates fluorine, chlorine, bromine or iodine.

With the term "cyano" we intend a -CN residue.

With the term "amino" we intend the group $-NH_2$.

With the term "nitro" we intend a $-NO_2$ group.
With the term "hydroxy" we intend the group -OH.
With the term "alkoxy" we intend any of the above $C_1$-$C_6$ alkyl groups linked to the rest of the molecule through a oxygen atom (-O-), such as methoxy, ethoxy, n-propoxy, isopropoxy and the like.
With the term "trifluoromethyl" we intend -CF3.
It is clear to the skilled person that any group which name is a composite name such as, for instance, arylalkyl has to be intended as conventionally construed by the parts from which it derives, e.g. by an alkyl group which is further substituted by aryl, wherein alkyl and aryl are as above defined. Examples of aryalkyl are benzyl ($-CH_2Ph$), phenylethyl ($-CH_2CH_2Ph$) and the like.
The term "pharmaceutically acceptable salt" of compounds of formula (I) refers to those salts that retain the biological effectiveness and properties of the parent compound. Such salts include:

acid addition salts with inorganic acids such as hydrochloric, hydrobromic, nitric, phosphoric, sulfuric, perchloric acid and the like, or with organic acids such as acetic, trifluoroacetic, propionic, glycolic, lactic, (D) or (L) malic, maleic, methanesulfonic, ethanesulfonic, benzoic, p-toluenesulfonic, salicylic, cinnamic, mandelic, tartaric, citric, succinic, malonic acid and the like; salts formed when an acidic proton present in a compound of formula (I) is either replaced by a metal ion, e.g., an alkali metal ion such as sodium or potassium, or an alkaline earth ion such as calcium or magnesium, or coordinates with an organic base such as ethanolamine, diethanolamine, triethanolamine, tromethamine, N-methylglucamine, and the like.

Compounds of formula (I) wherein:

**X** is -N(**R6**)-$CH_2$-, **A** is -$CH_2$-, **Y** is -CH= and **R6** is hydrogen or a group -CO**R7**,
are represented by the general formula (Ia):

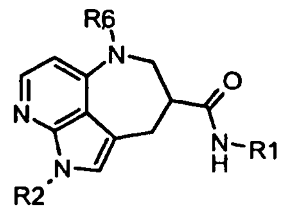

**(Ia)**

Compounds of formula (I) wherein:

**X** is -N(**R6**)$CH_2$-, **A** is -CH=, **Y** is -CH= and **R6** is hydrogen or a group -CO**R7**,

are represented by the general formula (Ib):

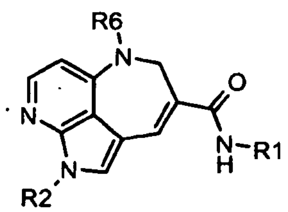

**(Ib)**

Compounds of formula (I) wherein:

**X** is -$CH_2$-$CH_2$, **A** is -CH= and **Y** is -CH=,

are represented by the general formula (Ic):

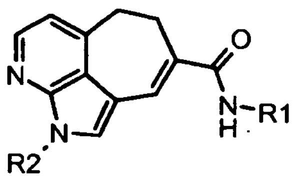

(Ic)

Compounds of formula (I) wherein:

**X** is -CH=, **A** is -CH= and **Y** is $-CH_2-$,

Are represented by the general formula (Id):

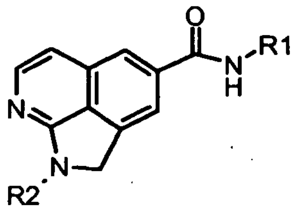

(Id)

A preferred class of compounds of the present invention is represented by the derivatives of formula (Ia) as defined above, wherein:

**R1** is as defined above,
**R2** is hydrogen or a group -CO**R7**;
**R7** is an optionally substituted straight or branched $C_1$-$C_6$ alkyl.

A more preferred class of compounds of formula (Ia) as defined above is represented by derivatives wherein:

**R1** is hydrogen or a group of formula (II) as above defined;
**R2** is hydrogen or a group -CO**R7**;
**R3** is an optionally substituted aryl or arylalkyl;
**R4** and **R5** are hydrogen or together with the nitrogen atom to which they are bonded form an optionally substituted pyrrolidino, morpholino, N-methylpiperazino or piperidino ring;
**R7** is methyl or phenyl.

A most preferred class of compounds of formula (Ia) as defined above is represented by derivatives wherein:

**R1** is hydrogen or a group of formula (II) as above defined;
**R2** is hydrogen;
**R3** is phenyl or benzyl;
**R4** and **R5** are hydrogen or together with the nitrogen atom to which they are bonded form an optionally substituted pyrrolidino ring;
**R7** is methyl.

Another preferred class of compounds of the present invention is represented by the derivatives of formula (Ib) as defined above, wherein:

**R1** is as defined above for the compound of formula (I),
**R2** is hydrogen or a group -CO**R7**;
**R6** is hydrogen or a group -CO**R7;**

**R7** is an optionally substituted straight or branched $C_1$-$C_6$ alkyl.

A more preferred class of compounds of formula (Ib) as defined above is represented by derivatives wherein:

**R1** is hydrogen or a group of formula (II) as defined above;
**R3** is an optionally substituted aryl or arylalkyl;
**R4** and **R5** are hydrogen or together with the nitrogen atom to which they are bonded form an optionally substituted pyrrolidino, morpholino, N-methylpiperazino or piperidino ring;
**R6** is hydrogen or a group -CO**R7**;
**R7** is methyl or phenyl.

A most preferred class of compounds of formula (Ib) as defined above is represented by derivatives wherein:

**R1** is hydrogen or a group of formula (II) as defined above;
**R2** is hydrogen;
**R3** is phenyl or benzyl;
**R4** and **R5** are hydrogen or together with the nitrogen atom to which they are bonded form an optionally substituted pyrrolidino ring;
**R6** is hydrogen or a group -CO**R7**;
**R7** is methyl.

A preferred class of compounds of the present invention is represented by the derivatives of formula (Ic) as defined above, wherein:

**R1** is as defined above for the compound of formula (I),
**R2** is hydrogen or a group -CO**R7**;
**R7** is an optionally substituted straight or branched $C_1$-$C_6$ alkyl.

A more preferred class of compounds of formula (Ic) as defined above is represented by derivatives wherein:

**R1** is hydrogen or a group of formula (II) as defined above;
**R2** is hydrogen or a group -CO**R7**;
**R3** is an optionally substituted aryl or arylalkyl;
**R4** and **R5** are hydrogen or together with the nitrogen atom to which they are bonded form an optionally substituted pyrrolidino, morpholino, N-methylpiperazino or piperidino ring;
**R7** is methyl or phenyl.

A most preferred class of compounds of formula (Ic) as defined above is represented by derivatives wherein:

**R1** is hydrogen or a group of formula (II) as defined above;
**R2** is hydrogen;
**R3** is phenyl or benzyl;
**R4** and **R5** are hydrogen or together with the nitrogen atom to which they are bonded form an optionally substituted pyrrolidino ring.

A preferred class of compounds of the present invention is represented by the derivatives of formula (Id) as defined above, wherein:

**R1** is as defined above for the compound of formula (I),
**R2** is hydrogen or a group -CO**R7**;
**R7** is an optionally substituted straight or branched $C_1$-$C_6$ alkyl.

A more preferred class of compounds of formula (Id) as defined above is represented by derivatives wherein:

**R1** is hydrogen or a group of formula (II) as defined above;
**R2** is hydrogen or a group -CO**R7**;
**R3** is an optionally substituted aryl or arylalkyl;
**R4** and **R5** are hydrogen or together with the nitrogen atom to which they are bonded form an optionally substituted

pyrrolidino, morpholino, N-methylpiperazino or piperidino ring;
**R7** is methyl or phenyl.

A most preferred class of compounds of formula (Id) is represented by derivatives wherein:

**R1** is hydrogen or a group of formula (II) as defined above;
**R2** is hydrogen;
**R3** is phenyl or benzyl;
**R4** and **R5** are hydrogen or together with the nitrogen atom to which they are bonded form an optionally substituted pyrrolidino ring.

Specific compounds (Cpd..) of the invention are listed below:

1. *N*-[(1*S*)-2-amino-1-benzylethyl]-6,7-dihydro-2*H*-2,3-diazabenzo[*cd*]azulene-8-carboxamide;
2. *N*-[(1*S*)-2-amino-1-benzylethyl]-6,7-dihydro-2*H*-2,3,6-triazabenzo[*cd*]azulene-8-carboxamide;
3. 6-acetyl-*N*-[(1*S*)-2-amino-1-benzylethyl]-6,7-dihydro-2*H*-2,3,6-triazabenzo[*cd*]azulene-8-carboxamide;
4. *N*-[(1*S*)-2-amino-1-benzylethyl]-6,7,8,9-tetrahydro-2*H*-2,3,6-triazabenzo[*cd*]azulene-8-carboxamide;
5. 6,7,8,9-tetrahydro-2*H*-2,3,6-triazabenzo[*cd*]azulene-8-carboxamide;
6. *N*-[(1*S*)-1-phenyl-2-pyrrolidin-1-ylethyl]-6,7-dihydro-2*H*-2,3-diazabenzo[*cd*]azulene-8-carboxamide;
7. *N*-[(1*S*)-2-amino-1-benzylethyl]-1,2-dihydropyrrolo[4,3,2-*ij*]isoquinoline-4-carboxamide;
8. *N*-[(1*S*)-1-phenyl-2-pyrrolidin-1-ylethyl]-1,2-dihydropyrrolo[4,3,2-*ij*]isoquinoline-4-carboxamide;
9. 6,7-dihydro-2H-2,3-diazabenzo[cd]azulene-8-carboxamide;
10. N-methyl-6,7-dihydro-2H-2,3-diazabenzo[cd]azulene-8-carboxamide;
11. N-benzyl-6,7-dihydro-2H-2,3-diazabenzo[cd]azulene-8-carboxamide;
12. N-(3-chlorobenzyl)-6,7-dihydro-2H-2,3-diazabenzo[cd]azulene-8-carboxamide;
13. N-(3-methoxybenzyl)-6,7-dihydro-2H-2,3-diazabenzo[cd]azulene-8-carboxamide;
14. N-[2-(dimethylamino)ethyl]-6,7-dihydro-2H-2,3-diazabenzo[cd]azulene-8-carboxamide;
15. N-(2-morpholin-4-ylethyl)-6,7-dihydro-2H-2,3-diazabenzo[cd]azulene-8-carboxamide;
16. N-[(1S)-2-morpholin-4-yl-1-phenylethyl]-6,7-dihydro-2H-2,3-diazabenzo[cd]azulene-8-carboxamide;
17. N-[(1S)-2-amino-1-phenylethyl]-6,7-dihydro-2H-2,3-diazabenzo[cd]azulene-8-carboxamide;
18. N-[(1S)-2-(4-methylpiperazin-1-yl)-1-phenylethyl]-6,7-dihydro-2H-2,3-diazabenzo[cd]azulene-8-carboxamide;
19. N-[(1S)-2-amino-1-(1H-indol-3-ylmethyl)ethyl]-6,7-dihydro-2H-2,3-diazabenzo[cd]azulene-8-carboxamide;
20. N-[(1S)-2-amino-1-(naphthalen-1-ylmethyl)ethyl]-6,7-dihydro-2H-2,3-diazabenzo[cd]azulene-8-carboxamide;
21. 6,7-dihydro-2H-2,3,6-triazabenzo[cd]azulene-8-carboxamide;
22. N-methyl-6,7-dihydro-2H-2,3,6-triazabenzo[cd]azulene-8-carboxamide;
23. N-benzyl-6,7-dihydro-2H-2,3,6-triazabenzo[cd]azulene-8-carboxamide;
24. N-(3-chlorobenzyl)-6,7-dihydro-2H-2,3,6-triazabenzo[cd]azulene-8-carboxamide;
25. N-(3-methoxybenzyl)-6,7-dihydro-2H-2,3,6-triazabenzo[cd]azulene-8-carboxamide;
26. N-[2-(dimethylamino)ethyl]-6,7-dihydro-2H-2,3,6-triazabenzo[cd]azulene-8-carboxamide;
27. N-(2-morpholin-4-ylethyl)-6,7-dihydro-2H-2,3,6-triazabenzo[cd]azulene-8-carboxamide;
28. N-[(1S)-2-morpholin-4-yl-1-phenylethyl]-6,7-dihydro-2H-2,3,6-triazabenzo[cd]azulene-8-carboxamide;
29. N-[(1S)-2-amino-1-phenylethyl]-6,7-dihydro-2H-2,3,6-triazabenzo[cd]azulene-8-carboxamide;
30. N-[(1S)-2-(4-methylpiperazin-1-yl)-1-phenylethyl]-6,7-dihydro-2H-2,3,6-triazabenzo[cd]azulene-8-carboxamide;
31. N-[(1S)-2-amino-1-(1H-indol-3-ylmethyl)ethyl]-6,7-dihydro-2H-2,3,6-triazabenzo[cd]azulene-8-carboxamide;
32. N-[(1S)-2-amino-1-(naphthalen-1-ylmethyl)ethyl]-6,7-dihydro-2H-2,3,6-triazabenzo[cd]azulene-8-carboxamide;
33. N-[(1S)-1-phenyl-2-pyrrolidin-1-ylethyl]-6,7-dihydro-2H-2,3,6-triazabenzo[cd]azulene-8-carboxamide;
34. 6-acetyl-6,7-dihydro-2H-2,3,6-triazabenzo[cd]azulene-8-carboxamide;
35. 6-acetyl-N-methyl-6,7-dihydro-2H-2,3,6-triazabenzo[cd]azulene-8-carboxamide;
36. 6-acetyl-N-benzyl-6,7-dihydro-2H-2,3,6-triazabenzo[cd]azulene-8-carboxamide;
37. 6-acetyl-N-(3-chlorobenzyl)-6,7-dihydro-2H-2,3,6-triazabenzo[cd]azulene-8-carboxamide;
38. 6-acetyl-N-(3-methoxybenzyl)-6,7-dihydro-2H-2,3,6-triazabenzo[cd]azulene-8-carboxamide;
39. 6-acetyl-N-[2-(dimethylamino)ethyl]-6,7-dihydro-2H-2,3,6-triazabenzo[cd]azulene-8-carboxamide;
40. 6-acetyl-N-(2-morpholin-4-ylethyl)-6,7-dihydro-2H-2,3,6-triazabenzo[cd]azulene-8-carboxamide;
41. 6-acetyl-N-[(1S)-2-morpholin-4-yl-1-phenylethyl]-6,7-dihydro-2H-2,3,6-triazabenzo[cd]azulene-8-carboxamide;
42. 6-acetyl-N-[(1S)-2-amino-1-phenylethyl]-6,7-dihydro-2H-2,3,6-triazabenzo[cd]azulene-8-carboxamide;
43. 6-acetyl-N-[(1S)-1-phenyl-2-pyrrolidin-1-ylethyl]-6,7-dihydro-2H-2,3,6-triazabenzo[cd]azulene-8-carboxamide;

44. 6-acetyl-N-[(1S)-2-(4-methylpiperazin-1-yl)-1-phenylethyl]-6,7-dihydro-2H-2,3,6-triazabenzo[cd]azulene-8-carboxamide;
45. 6-acetyl-N-[(1S)-2-amino-1-(1H-indol-3-ylmethyl)ethyl]-6,7-dihydro-2H-2,3,6-triazabenzo[cd]azulene-8-carboxamide;
46. 6-acetyl-N-[(1S)-2-amino-1-(naphthalen-1-ylmethyl)ethyl]-6,7-dihydro-2H-2,3,6-triazabenzo[cd]azulene-8-carboxamide;
47. 1,2-dihydropyrrolo[4,3,2-ij]isoquinoline-4-carboxamide;
48. N-methyl-1,2-dihydropyrrolo[4,3,2-ij]isoquinoline-4-carboxamide;
49. N-benzyl-1,2-dihydropyrrolo[4,3,2-ij]isoquinoline-4-carboxamide;
50. N-(3-chlorobenzyl)-1,2-dihydropyrrolo[4,3,2-ij]isoquinoline-4-carboxamide;
51. N-(3-methoxybenzyl)-1,2-dihydropyrrolo[4,3,2-ij]isoquinoline-4-carboxamide;
52. N-[2-(dimethylamino)ethyl]-1,2-dihydropyrrolo[4,3,2-ij]isoquinoline-4-carboxamide;
53. N-(2-morpholin-4-ylethyl)-1,2-dihydropyrrolo[4,3,2-ij]isoquinoline-4-carboxamide;
54. N-[(1S)-2-morpholin-4-yl-1-phenylethyl]-1,2-dihydropyrrolo[4,3,2-ij]isoquinoline-4-carboxamide;
55. N-[(1S)-2-amino-1-phenylethyl]-1,2-dihydropyrrolo[4,3,2-ij]isoquinoline-4-carboxamide;
56. N-[(1S)-2-(4-methylpiperazin-1-yl)-1-phenylethyl]-1,2-dihydropyrrolo[4,3,2-ij]isoquinoline-4-carboxamide;
57. N-[(1S)-2-amino-1-(1H-indol-3-ylmethyl)ethyl]-1,2-dihydropyrrolo[4,3,2-ij]isoquinoline-4-carboxamide and
58. N-[(1S)-2-amino-1-(naphthalen-1-ylmethyl)ethyl]-1,2-dihydropyrrolo[4,3,2-ij]isoquinoline-4-carboxamide.

For a reference to any specific compound of formula (I) of the invention, optionally in the form of a pharmaceutically acceptable salt, see the experimental section and claims. The present inventions also provides a process for the preparation of compounds of formula (I) as defined above, characterized in that the process comprises:

g) reacting a compound of formula (IXa), (IXb), (IXc) or (IXd):

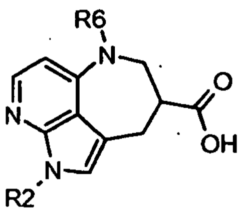

(IXa) OR

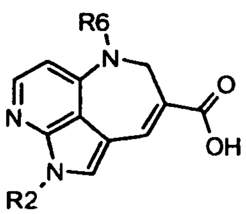

(IXb) OR

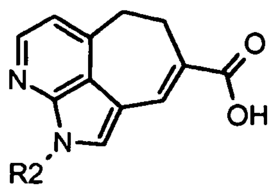

(IXc)

OR

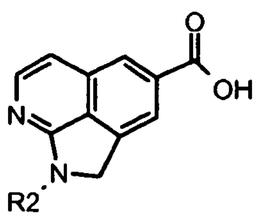

(IXd)

wherein **R2** and **R6** are as defined above, with an amine compound of formula (XIX):

**R1**-NH2 (XIX)

wherein **R1** is as defined above, to give a compound of formula (I), optionally separating the resulting compound into the single isomers, transforming the compound of formula (I) into a different compound of formula (I), and/or into a pharmaceutically acceptable salt if desired.

The present invention further provides a process for the preparation of a compound of formula (Ia) as defined above, characterized in that the compound of formula (IXa) as defined above, is prepared according to the following steps:

a) reacting 4-Iodo-1H-pyrrolo[2,3-b]pyridine with a compound of formula (XV):

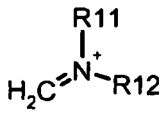

(XV)

wherein **R11** and **R12** are $C_1$-$C_6$ alkyl or taken together with the nitrogen atom to which they are bonded, form an optionally substituted heterocycloalkyl group;

b) reacting the resulting compound of formula (III):

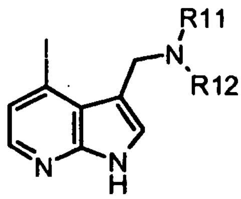

(III)

wherein **R11** and **R12** are as defined above, with a compound of formula (IV):

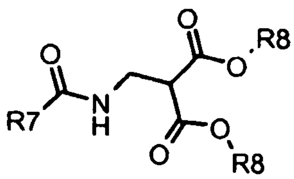

(IV)

wherein **R7** is as defined above and **R8** is $C_1$-$C_6$ alkyl;

c) hydrolyzing the resulting compound of formula (V):

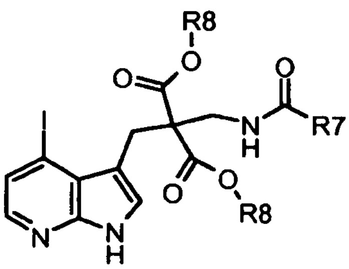

(V)

wherein **R7** and **R8** are as defined above;

d) esterifying the resulting compound of formula (VI):

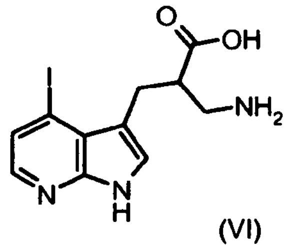

(VI)

with an alcohol of formula (XIV):

**R8**-OH (XIV)

wherein **R8** is as defined above;

e) cyclizing the resulting compound of formula (VII):

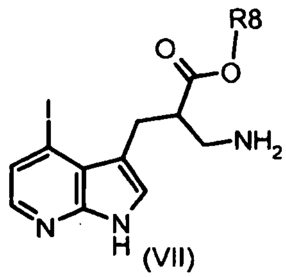

(VII)

wherein **R8** is as defined above;

f) hydrolyzing the resulting compound of formula (VIII):

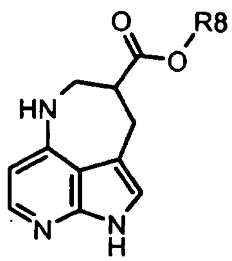

(VIII)

wherein **R8** is as defined above to give a compound of formula (IXa) wherein **R2** and **R6** are hydrogen.

The present invention further provides a process for the preparation of a compound of formula (Ia) as defined above, characterized in that the compound of formula (IXa) as defined above, is prepared according to the following steps:

h) reacting the compound of formula (VIII) as defined above, with the compounds of formula (XI):

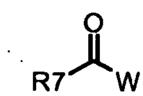

(XI)

wherein W is hydroxyl, halogen or a suitable leaving group and **R7** is as defined above;

i) hydrolyzing of the resulting compound of formula (XII):

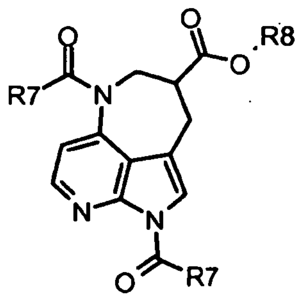

(XII)

wherein **R7** and **R8** are as defined above, to give a compound of formula (IXa) wherein **R2** is hydrogen and **R6** is a group -CO**R7**, wherein **R7** is as defined above.

The present invention further provides a process for the preparation of a compound of formula (Ib) as defined above, characterized in that the compound of formula (IXb) as defined above, is prepared according to the following steps:

j) oxidizing the compound of formula (XII) as defined above;
k) hydrolyzing the resulting compound of formula (XIII):

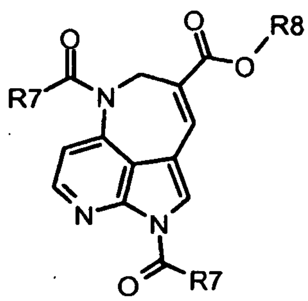

(XIII)

wherein **R7** and **R8** are as defined above, to give a compound of formula (IXb) wherein **R2** is hydrogen and **R6** is a group -CO**R7**, wherein **R7** is as defined above.

The present invention further provides a process for the preparation of a compound of formula (Ib) as defined above, characterized in that the compound of formula (IXb) as defined above, is prepared according to the following steps:

l) hydrolyzing the compound of formula (IXb) wherein **R2** is hydrogen and **R6** is a group -CO**R7,** wherein **R7** is as defined above, to give a compound of formula (IXb) wherein **R2** and **R6** are hydrogen.

The present invention further provides a process for the preparation of a compound of formula (Ic) and (Id) as defined above, characterized in that the compound of formula (IXc) and (IXd) as defined above, are prepared according to the following steps: m) protecting 4-Iodo-1H-pyrrolo[2,3-b]pyridine with a compound of formula (XVI):

**R9-J** (XVI)

wherein **R9** is a suitable protecting group and **J** is halogen or a suitable leaving group;

n) reacting the resulting compound of formula (XX):

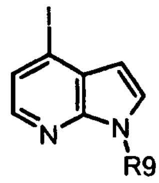

(XX)

wherein **R9** is as defined above, with a compound of formula (XVII):

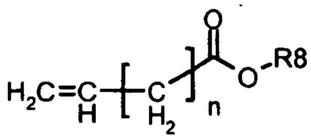

XVII

wherein **n** is 0 or 1, and **R8** is as defined above; o) reducing the resulting compound of formula (XXI):

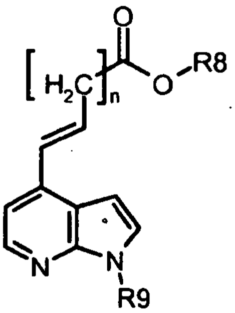

(XXI)

wherein **n**, **R8** and **R9** are as defined above; p) deprotecting the resulting compound of formula (XXII):

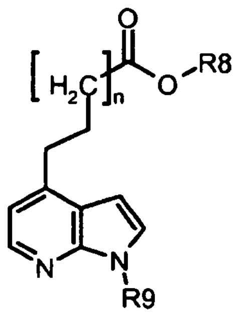

(XXII)

wherein **n**, **R8** and **R9** are as defined above; q) formylating the resulting compound of formula (XXIII):

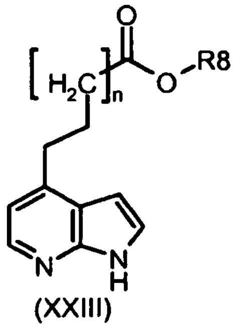

(XXIII)

wherein **n** and **R8** are as defined above, with a formylating agent; r) hydrolyzing the resulting compound of formula (XXIV):

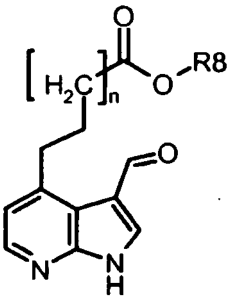

(XXIV)

wherein **n** and **R8** are as defined above; s) cyclocondensing intramolecularly the resulting compound of formula (XXV):

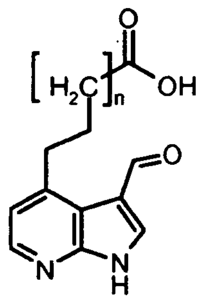

(XXV)

wherein **n** is as defined above, in presence of a compound of formula (XI):

**R7**-CO-W (XI)

wherein **R7** and **W** are as defined above;
t) hydrolyzing the resulting compound of formula (XXVI):

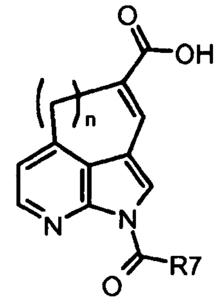

(XXVI)

wherein **n** is 1 or 2 and **R7** are as defined above, to give a compound of formula (IXc) or (IXd) wherein **R2** is hydrogen.

The present invention further provides a process for the preparation of a compound of formula (I) as defined above, characterized in that the compound of formula (IXa), (IXb), (IXc) or (IXd) as defined above are prepared according to the following steps:

u) reacting a compound of formula (IXa) or (IXb) wherein **R2** is hydrogen and **R6** is as defined above, or a compound of formula (IXc) or (IXd) wherein **R2** is hydrogen, with a compound of formula (XIV) as defined above;
v) reacting the resulting compound of formula (XXVIIa), (XXVIIb), (XXVIIc) or (XXVIId):

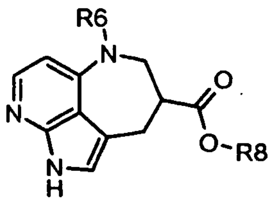

(XXVIIa) OR

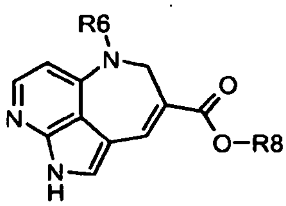

(XXVIIb) OR

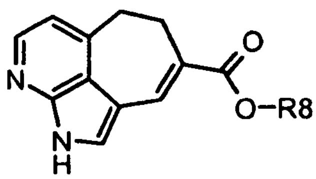

(XXVIIc)

OR

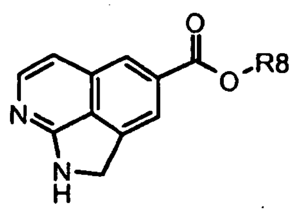

(XXVIId)

wherein **R6** and **R8** are as defined above, with a compound of formula (XXVIII):

**R2**-L (XXVIII)

wherein **R2** is as defined above but not hydrogen, and L is halogen or a suitable leaving group;
w) hydrolysing the resulting compound of formula (XXIXa), (XXIXb), (XXIXc) or (XXIXd):

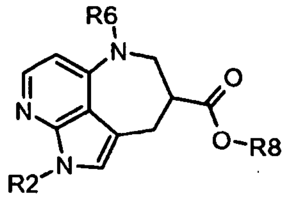

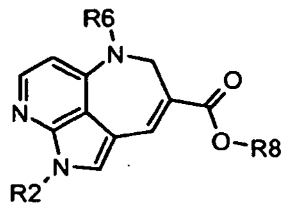

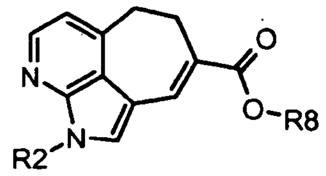

(XXIXa) OR (XXIXb) OR (XXIXc)

OR

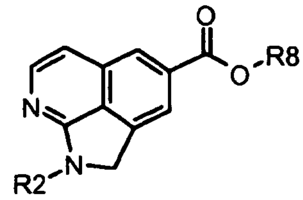

(XXIXd)

wherein **R2** is as defined above but not hydrogen and **R8** is as defined above, to give a compound of formula (IXa), (IXb), (IXc) or (IXd) wherein **R2** is as defined above but not hydrogen.

A compound of formula (I) can be transformed into another compound of formula (I) for example by deprotection.

A compound of formula (I) can also be transformed into a pharmaceutically acceptable salt according to standard procedures that are known to those skilled in the art. Alternatively, a compound of formula (I) that is obtained as a salt can be transformed into the free base or the free acid according to standard procedures that are known to the skilled person.

The synthesis of a compound of formula (I), according to the synthetic process described above, can be accomplished in a stepwise manner, whereby each intermediate is isolated and purified by standard purification techniques, like, for example, column chromatography, before carrying out the subsequent reaction. Alternatively, two or more steps of the synthetic sequence can be carried out in a so-called "one-pot" procedure, as known in the art, whereby only the compound resulting from the two or more steps is isolated and purified.

According to the step a) of the process, the reaction of 4-Iodo-1H-pyrrolo[2,3-b]pyridine with the compound of formula (XVI) to give the compound of formula (III) can be accomplished in a variety of ways and experimental conditions, which are widely known in the art as Mannich reaction. For example, the reaction is carried out with a synthetic equivalent of formaldehyde such as paraformaldehyde and an amine such as dimethylamine hydrochloride that generated in situ the compound of formula (XV) in a suitable solvent such as methanol, ethanol, or n-butanol at a temperature ranging from 60°C to reflux, for a time ranging from 30 min. to about 4 hours.

According to the step b) of the process, the reaction of the compound of formula (III) with the diethylmalonate derivative of formula (IV) to give the compound of formula (V) can be carried out in a variety of ways and experimental conditions. For example, the reaction is performed in the presence of a base such as lithium, sodium or potassium hydrate in a suitable solvent such as toluene, xylene at reflux for a time ranging from 1 to 4 hours.

According to the step c) of the process, the hydrolysis of the compound of formula (V) to give the compound of formula (VI) can be carried out in a variety of ways and experimental conditions. For example, it is performed in the presence of an acid such as aqueous concentrated hydrochloric acid at reflux for 8 hours.

According to the step d) of the process, the esterification of the compound of formula (VI) to give the compound of formula (VII) can be accomplished in a variety of ways and experimental conditions, which are widely known in the art as ester formation. For example, the reaction is performed in the presence of suitable mixture of solvent such as ethanol and toluene in the presence of an acidic catalyst such as p-toluensulfonic acid at reflux for a time varying from about 30 min. to about 24 hours.

According to the step e) of the process, the cyclization of the compound of formula (VII) into the compound of formula (VIII) can be carried out in a variety of ways and experimental conditions. For example, it is carried out at a temperature of about 180°C for a time ranging from about 30 min. to about 4 hours, in a suitable solvent such as

diphenylether.

According to the step f) of the process, the hydrolysis of the compound of formula (VIII) to give the compound of formula (IXa) can be accomplished in a variety of ways and experimental conditions, which are widely known in the art as ester hydrolysis. As an example, this reaction is carried out under basic conditions, for instance in the presence of an inorganic base such as sodium hydrate or potassium hydrate or lithium hydrate in a suitable solvent such as ethanol or a mixture of tetrahydrofurane (THF) methanol and water at room temperature for a time ranging from 1 to 24 hours.

According to the step g) of the process, the reaction of the compound of formula (IXa), (IXb), (IXc) or (IXd) with a compound of formula (XIX) to give the compound of formula (I) can be accomplished in a variety of ways and experimental conditions, which are widely known in the art for the preparation of amides. For example, the reaction of the carboxylic acid compound of formula (IX) with amines and in particular the compounds of formula (II) is carried out in the presence of a suitable condensing agent such as 2-(1H-benzotriazol-1-yl)-1,1,3,3-tetramethyluronium tetrafluoroborate (TBTU), soluble or polymer supported 1,3-dicyclohexylcarbodiimide (DCC), 1,3-diisopropylcarbodiimide (DIC), 1-(3-dimethylaminopropyl)-3-ethylcarbodiimide (EDCI), 1.1'-carbonyldiimidazole (CDI), benzotriazol-1-yloxy-tripyrrolidinophosphonium hexafluorophosphate (PyBOP), in a suitable solvent such as, for instance, dichloromethane, chloroform, tetrahydrofuran, diethyl ether, 1,4-dioxane, acetonitrile, toluene, dimethylacetamide (DMA), N-methylpyrrolidone (NMP), or N,N-dimethylformamide (DMF), at a temperature ranging from about -10°C to reflux and for a suitable time, for instance from about 30 minutes to 96 hours. The said reaction is optionally carried out in the presence of a suitable catalyst, for instance 4-dimethylaminopyridine (DMAP), or in the presence of a further coupling reagent such as N-hydroxybenzotriazole (HOBT), or in the presence of a suitable base such as triethylamine (TEA) or N,N-diisopropyl-N-ethylamine (DIPEA).

Alternatively, this same reaction is also carried out, for example, through a mixed anhydride method, by using an alkyl chloroformate such as ethyl, iso-butyl, or isopropyl chloroformate, in the presence of a tertiary base such as triethylamine, N,N-diisopropyl-N-ethylamine or pyridine, in a suitable solvent such as, for instance, toluene, dichloromethane, chloroform, tetrahydrofuran, acetonitrile, diethyl ether, 1,4-dioxane, or N,N-dimethylformamide, at a temperature ranging from about -30°C to room temperature.

According to the step h) of the process, the reaction of the compound of formula (VIII) with the acylating agent of formula (XI) to give the compound of formula (XII) can be carried out in a variety of ways and experimental conditions. For example, the reaction is performed in a suitable solvent such as chloroform, dichloromethane, diethylether, tetrahydrofuran or 1,4-dioxane at a temperature ranging from room temperature to reflux for a time ranging from 2 to 6 hours.

According to the step i) of the process, the hydrolysis of the compound of formula (XII) to give the compound of formula (IXa) can be carried out in a way analogous to that specified above under f).

According to the step j) of the process, the oxidation of the compound of formula (XII) to give a compound of formula (XIII) can be accomplished in a variety of ways according to conventional methods for oxidation. For example, this reaction is carried out in the presence of a suitable oxidizing agent such as 2,3-dichloro-5,6-dicyano-benzoquinone (DDQ) in a suitable solvent such as dichloromethane at room temperature to reflux and for a suitable time, for instance from about 1 hour to about 48 hours.

According to the step k) of the process, hydrolysis of the compound of formula (XIII) to give a compound of formula (IXb) can be accomplished in a variety of ways according to conventional methods for hydrolysis. For example, it is carried out in the presence of an inorganic base such as lithium, sodium or potassium hydrate in a suitable solvent such as methanol or ethanol at room temperature for a time of about 20 hours. According to the step 1) of the process, the hydrolysis of the compound of formula (IXb) to give another compound of formula (IXb) can be accomplished in a variety of ways according to conventional methods for hydrolysis. For example, it is carried out in the presence of an organic or inorganic acid such as hydrogen choride, or trifluoroacetic acid in a suitable solvent such as 1,4-dioxane at a temperature ranging from room temperature to reflux for a time ranging from 1 to 6 hours.

According to the step m) of the process, the protection of a compound of formula (X) with a compound of formula (XVI) can be accomplished in a variety of ways and experimental conditions, which are widely known in the art for the insertion of a protecting group. For example, when R9 is a triisopropylsilyl (TIPS) the reaction is carried out with triisopropylsilyl chloride (TIPS-C1), in the presence of a base such as sodium bis(trimethylsilyl)amide in a suitable solvent such as diethylether, tetrahydrofuran or 1,4-dioxane at a temperature of 0°C for a time of about 30 minutes. Alternatively, when R9 is a dimethyl-*tert*-butylsilyl the reaction is carried out with dimethyl-*tert*-butylsilyl chloride (TBDMS-C1), in the presence of a base such as sodium bis(trimethylsilyl)amide in a suitable solvent such as diethylether, tetrahydrofuran or dioxane at a temperature of 0°C for a time of about 30 minutes.

According to the step n) of the process, the reaction of the compound of formula (XX) with the compound of formula (XVII) to give the compound of formula (XXI) can be accomplished in a variety of ways and experimental conditions, which are widely known in the art as Heck reaction. For example, this reaction is carried out in the presence of palladium catalyst such as palladium acetate, with a phosphine such as tris(o-tolyl)-phosphine in presence of potassium carbonate, or of a tertiary amine such as triethylamine (TEA), and in a suitable solvent such as tetrahydrofuran, 1,4-dioxane, acetonitrile at reflux for a time varying from about 30 min. to about 24 hours.

According to the step o) of the process, the reduction of the compound of formula (XXI) into a compound of formula (XXII) can be accomplished in a variety of ways and experimental conditions, which are widely known in the art as double bond reduction. For example, this reaction is carried out with hydrogen in the presence of a suitable catalyst such as palladium on charcoal 5 %, or 10% in a suitable solvent such as ethanol at a pressure of about 30 psi for a time varying from about 30 min. to about 24 hours. Alternatively, the reaction is carried out in the presence of a reducing agent such as sodium borohydride and a catalyst such as bismuth trichloride in a suitable solvent such as ethanol at a temperature of about 0°C for a time varying from about 30 min. to about 4 hours.

According to the step p) of the process, the deprotection of the compound of formula (XXII) to give the compound of formula (XXIII) can be accomplished in a variety of ways and experimental conditions, which are widely known in the art as protecting group removal. For Example, the transformation is accomplished with tetrabutylammonium fluoride (TBAF) in a suitable solvent such as tetrahydrofuran at room temperature for a time of about 30 minutes.

According to the step q) of the process, the formylation of the compound of formula (XXIII) to give the compound of formula (XXIV) can be carried out in a variety of ways and experimental conditions. For example, the formylating agent is dichloromethyl methylether, in the presence of aluminium trichloride in a suitable solvent such as a mixture of nitromethane and dichloromethane at a temperature of about 0°C for a time of about 24 hours. Alternatively the formylating agent is hexamethylentetramine in a suitable solvent such as acetic acid at a temperature of about 100 °C for a time of about 2 hours.

According to the step r) of the process, the hydrolysis of the compound of formula (XXIV) to give the compound of formula (XXV) can be accomplished in a way analogous to that specified above under f).

According to the step s) of the process, the intramolecular cyclocondensation of the compound of formula (XXV) into a compound of formula (XXVI) can be accomplished in a variety of ways which are widely known in the art as Perkin condensation. Preferably, this reaction is carried out by reacting an aldehyde group with acetic acid derivative, as the compound of formula (XXV) possess both groups in proximity, the reaction occured intramolecularly, generating a new cycle. The reaction is performed in the presence of acetic anhydride and triethyl amine(TEA) which are also used as solvent at a temperature ranging from about room temperature to reflux and for a suitable time, for instance from about 1 hour to about 24 hours.

According to the step t) of the process, the hydrolysis of the compound of formula (XXVI) into the compound of formula (IX) can be accomplished in a way analogous to that specified above under f).

According to the step u) of the process, the reaction of the compound of formula (IXa), (IXb), (IXc) or (IXd) with the compound of formula (XIV) can be accomplished in a variety of ways and experimental conditions, which are widely known in the art as ester formation. For example, the reaction is performed in the presence of suitable mixture of solvent such as ethanol and toluene in the presence of an acidic catalyst such as p-toluensulfonic acid at reflux for a time varying from about 30 min. to about 24 hours.

According to the step v) of the process, the reaction of the compound of formula (XXVIIa), (XXVIIb), (XXVIIc) or (XXVIId) with the compound of formula (XXVIII) can be carried out in a variety of ways and experimental conditions. For example, the reaction is performed with straight or branched $C_1$-$C_6$ alkyl or aryalkyl bromide in the presence of suitable solvent such as N,N-dimethylacetamide, N,N-dimethylformamide or 1,4-dioxane in the presence of a base such as sodium carbonate or potassium carbonate or sodium hydride at a temperature ranging from about 0°C to room temperature for a suitable time for instance from about 30 minutes to 24 hours. According to the step w) of the process, the hydrolysis of the compound of formula (XXIXa), (XXIXb), (XXIXc) or (XXIXd) can be accomplished in a way analogous to that specified above under f).

A compound of formula (I) when R1 is a group of formula (II) wherein R4 or R5 is a protected group of the amine moiety, can be transformed into a different compound of formula (I) wherein R4 and R5 are hydrogen, in a variety of ways according to conventional methods for deprotecting amino groups. Depending on the amino protecting group, this reaction can be conducted in different ways. In one aspect, such reaction can be carried out by treatment with an inorganic acid, such as hydrochloric, sulphuric or perchloric acid, or an organic acid, such as trifluoroacetic or methanesulfonic acid, in a suitable solvent, such as water, methanol, ethanol, 1,4-dioxane, tetrahydrofuran, diethyl ether, diisopropyl ether, acetonitrile, N,N-dimethylformamide, dichloromethane or mixtures thereof, at a temperature ranging from -10°C to 80°C, and for a period of time ranging from 30 minutes to 48 hours.

A compound of formula (I) can also be transformed into a pharmaceutically acceptable salt according to standard procedures that are known to those skilled in the art. Alternatively, a compound of formula (I) that is obtained as a salt can be transformed into the free base or the free acid according to standard procedures that are known to the skilled person.

4-Iodo-1H-pyrrolo[2,3-b]pyridine is prepared according to the procedure reported in WO2001002369A2.

The compounds of formula (IV) such as diethyl(2-acetylaminomethyl)malonate involved in the step b) are prepared following the process described by Cohen, S.G., et al in J. Am. Chem. Soc. 1961, 83, 4225-28.

The compounds of formula (XI), (XIV), (XVI), (XVII), (XIX) and (XXVII) are commercial available.

It is known to the skilled person that transformation of a chemical function into another may require that one

or more reactive centers in the compound containing this function be protected in order to avoid undesired side reactions. Protection of such reactive centers, and subsequent deprotection at the end of the synthetic transformations, can be accomplished following standard procedures described, for instance, in: Green, Theodora W. and Wuts, Peter G.M. - Protective Groups in Organic Synthesis, Third Edition, John Wiley & Sons Inc., New York (NY), 1999.

Those skilled in the art will recognize if a stereocenter exists in compounds of formula (I).

Accordingly, the present invention includes both possible stereoisomers and includes not only racemic compounds but the individual enantiomer as well. When a compound is desired as a single enantiomer, it may be obtained by stereospecific synthesis or by resolution of the final product or any convenient intermediate.

In cases where a compound of formula (I) contains one or more asymmetric centers, said compound can be separated into the single isomer by procedures known to those skilled in the art. Such procedures comprise standard chromatographic techniques, including chromatography using a chiral stationary phase, or crystallization. Stereochemistry of Organic Compounds by E.L.Eliel, S.H.Wilen and L.N. Mander (Wiley-Interscience, 1994*),* reports, for instance, general methods for separation of compounds containing one or more asymmetric centers.

The compounds of this invention may be made by a variety of methods, including standard chemistry. Any previously defined variable will continue to have the previously defined meaning unless otherwise indicated. Illustrative general synthetic methods are set out below and then the specific compounds of the invention are prepared in the Working Examples.

The compounds of this invention can be prepared from readily available starting materials using the following general methods and procedures. It will be appreciated that, where typical or preferred process conditions (i.e., reaction temperatures, times, mole ratios of reactants, solvents, pressures) are given, other process conditions can also be used unless otherwise stated. Optimum reaction conditions may vary with the particular reactants or solvent used, but one skilled in the art can determine such conditions by routine optimization procedures.

## PHARMACOLOGY

The short forms and abbreviations used herein have the following meaning:

| | |
|---|---|
| Ci | Curie |
| DMSO | dimethylsulfoxide |
| ID | identity |
| KDa | kiloDalton |
| microCi | microCurie |
| mg | milligram |
| microg | microgram |
| mL | milliliter |
| microL | microliter |
| μL | microliter |
| M | molar |
| mM | millimolar |
| microM | micromolar |
| nM | nanomolar |

### Preparation of AKT 1, 2 and 3 for use in biochemical assay

The N-terminal GST fusion proteins used for the assays were prepared according to the methods described in Kumar, C. C. Biochem. Biophys. ACTA 2001, 1526, 257-268, and in Alessi, D. R.. Biochem. J. 1998 331, 299-308.

### Assays

Compounds of the present invention were tested in biochemical as well as in cell-based assays, as described below.

### Biochemical assay for inhibitors of AKT1 kinase activity

The inhibitory activity of putative kinase inhibitors and the potency of selected compounds were determined using a trans-phosphorylation assay.

A specific substrate was incubated with the kinase in appropriate buffer conditions in the presence of ATP traced with $^{33}P$-γ-ATP (gamma phosphate-labeled, Redivue™ Code Number AH9968, 1000-3000Ci/mmole, Amersham

Biosciences Piscataway, NJ, USA), optimal cofactors and test compound.

At the end of the phosphorylation reaction, more than 98% cold and radioactive ATP were captured by an excess of Dowex ion exchange resin. The resin was allowed to settle to the bottom of reaction wells by gravity. Supernatant, containing substrate peptide, was subsequently withdrawn and transferred into a counting plate, and radioactivity (corresponding to phosphate incorporated into peptide) was evaluated by β-counting.

**Reagent/assay conditions**

*i. Dowex resin preparation*

500 g of wet resin (SIGMA, custom prepared resin DOWEX 1x8 200-400 mesh, 2.5 Kg) are weighed out and diluted to 2 1 in 150 mM sodium formate, pH 3.00.

The resin is allowed to settle down (some hours) and then the supernatant is discarded. After three washes as above over a couple of days, the resin is allowed to settle, the supernatant is discarded and two volumes of 150 mM sodium formate buffer are added per volume of pellet. The final pH was then measured and should be around 3.00.

The washed resin was kept at 4° C before use, and was stable for more than one week.

ii. Kinase Buffer (KB)

Kinase buffer was composed of 50 mM TRIS pH 7.5 containing10 mM $MgCl_2$, 1 mM DTT, 3 microM $Na_3VO_4$, and 0.2 mg/mL BSA. 3X KB is buffer of the same composition and pH as KB, but with three times the concentration of each component. iii.Assay conditions (final concentrations) for AKT1.

The kinase assay was run with a final enzyme concentration of 5 nM, in the presence of 132 microM ATP, 0.88 nM $^{33}$P-γ-ATP and 30 microM substrate, a carboxy-terminally biotinylated peptide of the following sequence: ARKRERTYSFGHHA-biotin. The peptide was obtained in batches of >95% peptide purity from PRIMM labs Inc. (Boston MA, USA).

Robotized dowex assay

The "test" mixture of 15 microL consisting of:

1) 5 μl/well of 3x Enzyme mix (done in Kinase Buffer 3X),
2) 5 μl/well of 3x substrate and ATP mix (done in ddH2O), together with $^{33}$P-γ-ATP,
3) 5 μl/well of 3x test compounds (diluted into ddH2O - 3% DMSO).

Dilution of compounds and assay scheme is reported below.

i. Dilution of compounds

10 mM stock solutions of test compounds in 100% DMSO were distributed into 96 well 12x8 format microtiter plates.

For % inhibition studies, dilution plates at 1 mM, 10 microM and 100 microM were prepared in 100% DMSO, then diluted to 3X final desired concentration (30, 3 and 0.3 microM) in $ddH_2O$, 3% DMSO. A Multimek 96 (Beckman Coulter, Inc. 4300 N. Harbor Boulevard, P.O. Box 3100 Fullerton, CA 92834-3100 USA) was used for compound pipetting into test plates.

For $IC_{50}$ determination, starting solutions of 30 microM compound in 3% DMSO were derived from 1 mM/100% DMSO stock solutions. These 30 microM starting solutions were used for generation of a further 9 serial 1/3 dilutions in $ddH_20$, 3% DMSO, so as to generate a 10-point dilution curve at 3X the final assay concentration. Serial dilution was conducted in 96-well plates using a Biomek 2000 (Beckman Coulter) system. Dilution curves of 7 compounds/plate were prepared, and each plate also included a 10-point dilution curve of Staurosporine, as well as several negative and positive control wells.

**Assay scheme**

384-well plates, V bottom (test plates) are prepared with 5 μl of the compound dilution (3X) and then placed onto a PlateTrak 12 robotized station (Perkin Elmer; the robot has one 384-tips pipetting head for starting the assay plus one 96-tips head for dispensing the resin) together with one reservoir for the Enzyme mix (3X) and one for the ATP mix (3X).

At the start of the run, the robot aspirates 5 μl of ATP mix, makes an air gap inside the tips (3 μl) and aspirates

5 μl of AKT mix. The following dispensation into the plates allows the kinase reaction to start upon 3 cycles of mixing, done by the robot itself.

At this point, the correct concentration is restored for all reagents.

The robot incubates the plates for 60 minutes at room temperature, and then stops the reaction by pipetting 70 μl of dowex resin suspension into the reaction mix. Three cycles of mixing are done immediately after the addition of the resin.

The resin suspension has to be carefully stirred during the whole step of reaction stop because its settling velocity is extremely high.

The resin suspension is very dense; in order to avoid tip clogging, wide bore tips are used to dispense it.

Another mixing cycle is performed after all the plates are stopped, this time using normal tips: the plates are then allowed to rest for about one hour in order to maximize ATP capture. At this point, 20 μl of the supernatant are transferred into 384-Optiplates (Perkin-Elmer), with 70 μl of Microscint 40 (Perkin-Elmer); after 5 min of orbital shaking the plates are read on a Perkin-Elmer Top Count radioactivity counter.

Data are analysed by an internally customized version of the SW package "Assay Explorer" that provides either % inhibition for primary assays or sigmoidal fittings of the ten-dilutions curves for $IC_{50}$ determination, for the secondary assays/hit confirmation routines.

**iii.** Data analysis

Data were analysed using a customized version of the "Assay Explorer" software package (Elsevier MDL, San Leandro, CA 94577). For single compound concentrations, inhibitory activity was typically expressed as % inhibition obtained in presence of compound, compared to total activity of enzyme obtained when inhibitor is omitted.

Compounds showing desired inhibition were further analysed in order to study the potency of the inhibitor through $IC_{50}$ calculation. In this case, inhibition data obtained using serial dilutions of the inhibitor were fitted by non-linear regression using the following equation:

$$v = v_0 + \frac{(v_0 - v_b)}{1 + 10^{n(\log IC_{50} - \log[I])}}$$

where $v_b$ is the baseline velocity, $v$ is the observed reaction velocity, $v_o$ is the velocity in the absence of inhibitors, and [I] is the inhibitor concentration.

**Cell-based assays for inhibitors of AKT1 kinase activity**

**Inhibition of AKT induced S6 ribosomal protein phosphorylation in MCF7**

MCF-7 cells (ATCC# HTB-22) were seeded in 96-well poly-lysine coated clear- and flat-bottomed black plates (Matrix Technologies Inc., Hudson, NH, USA), in E-MEM medium (MEM+ Earle's BSS + 2 mM glutamine + 0.1 mM non-essential amino acids) containing 10% of FCS at a density of 7000 cells/well, and incubated overnight at 37°C, 5% CO2, 100% relative humidity. After this incubation, cells were treated with increasing compound doses for 4 hour at 37°C. Afterwards, cells were fixed with PBS/3.7% paraformaldehyde for 15 min at room temperature, wash twice with 200 μl/well D-PBS and permeabilize with a D-PBS solution containing 0.1% Triton X-100 (Sigma-Aldrich, St. Louis, MO, USA) and 0.1 % powder Milk for 15 minutes (staining solution). To the wells was then, add the primary anti-phospho-S6 (Ser 235/236) antibody (Cell Signaling, cat. # 2211) diluted 1/200 in staining solution (50 μl/well) and incubate for 1 hour at 37°C.Wells were then washed twice with 200 μl/well D-PBS and add the anti-rabbit Cy™5-conjugated (Far-red) secondary antibody (Amersham Biosciences, Little Chalfont, Buckinghamshire, UK) (1/500) in staining solution (50 μl/well) containing 2 μg/ml DAPI (4,6-diamidino-2-phenylindole) and incubate for 1 hour at 37°C. Wells were then washed X2 with 200 μL/well D-PBS, and 200 microL PBS are left in each well for immunofluorescence analysis. Fluorescence images in the DAPI and Cy5™ channels were automatically acquired, stored and analysed using a Cellomics ArrayScan™ IV instrument (Cellomics, Pittsburgh, USA); the Cellomics Cytotoxicity Algorithm was used to quantify cytoplasmic fluorescence associated with phospho-S6 (Cy5™ signal parameter: "Mean Lyso Mass-pH") for each cell in 10 fields/well, and eventually expressed as a mean population value. Unless otherwise stated, reagents were obtained from Sigma-Aldrich, St. Louis, MO, USA.

**Biochemical assay for inhibitors of AKT2 kinase activity**

Assay conditions

The in vitro kinase inhibition assay was conducted in the same way as described for AKT1. The kinase assay was run with a final enzyme concentration of 10 nM, in the presence of 396 microM ATP, 0.88 nM $^{33}$P-γ-ATP and 8.5 microM substrate, a carboxy-terminally biotinylated peptide of the following sequence: ARKRERTYSFGHHA-biotin. The peptide was obtained in batches of >95% peptide purity from PRIMM labs Inc. (Boston MA, USA).

**Biochemical assay for inhibitors of AKT3 kinase activity**

Assay conditions

The in vitro kinase inhibition assay was conducted in the same way as described for AKT1. The kinase assay was run with a final enzyme concentration of 1 nM, in the presence of 180 microM ATP, 0.88 nM $^{33}$P-γ-ATP and 28.5 microM substrate, a carboxy-terminally biotinylated peptide of the following sequence: ARKRERTYSFGHHA-biotin. The peptide was obtained in batches of >95% peptide purity from PRIMM labs Inc. (Boston MA, USA). Most compounds of formula (I) of the present invention showed $IC_{50}$ values on AKT1, AKT2, AKT3 in the low micromolar range.

In addition the selected compounds have been characterized for specificity on a panel of many other kinases, among which PKAα, SGK1, Cdk2A, IGF1-R, Aurora-2, PLK1, SULU1, ERK2, CK2, GSK3β, PKCβ, VEGFR3, PDGFR.

**FABS-NMR screening**

The biochemical functional NMR screening on the proteins kinase AKT-1 and PKA was performed using the 3-FABS (**3-F**luorine **A**toms for **B**iochemical **S**creening) technique developed by C. Dalvit and the NMR group in house (C. Dalvit et al 2003 J. Am. Chem. Soc. 125 (47): 14620).

The method require the use of a $^{19}$F-labelled substrate. The same commercial peptide was used as substrate in both enzymatic reaction: the Tyr just before the phosphorilation site in the AKTide peptide was mutated in a 3-tryfluormethyl-Phe by Bachem.

The AKTide like peptide sequence is reported below:

H-Ala-Arg-Lys-Arg-Glu-Arg-Ala-F(3-CF3)-Ser-Phe-Gly-His-His-Ala-OH

The active human Ser-Thr protein kinase AKT-1 Full Length was expressed as GST fusion protein in insect cells and then the GST was cleaved with PreScission protease. The active cycled AMP-dependent human protein kinase A PKA was expressed as polyHis-tag protein in E.Coli and purified by affinity column.

Reactions were performed in an end point format in eppendorf of 0.5 ml using the buffer solution 50mM Tris, pH 7.5, 5mM $MgCl_2$, 1mM DTT, 8% D2O and 0.001% Triton-X-100; the final volume was of 560 μl. The substrate and protein concentration were of 30 μM and 25 nM respectively. The reactions were performed using an ATP concentration of about 9 times its $K_M$: 150 μM in the test with PKA and 1.5 mM in the AKT-1 experiments. A sample in absence of inhibitor was run as control in order to follow the ongoing of the reaction and it represents the 0% inhibition. Different concentration compound stock solutions in DMSOd6 were made in order to add the same amount of DMSO*d6* in each sample: usually the compounds stock were of 0.03, 0.2, 2, and 10 mM. 0.56 μl of these solution was added in each sample in order to reach the final compounds concentration of 30 nM, 200 nM 2 μM and 10 μM respectively; 0.56 μl of DMSO*d6* was added in the control too. The reactions were quenched after about 100/120 minutes with 10 mM of Staurosporine (Sigma code:S4400), transferred into the 5 mm NMR tube (Cortec code: NE-MP5-8) and put onto the Sample Management System (SMS) autosampler for automatic data collection. The spectra were recorded using the Varian Inova 600 MHz NMR spectrometer operating at a $^{19}$F Larmor frequency of 564 MHz and using a 5mm probe with the inner coil tuned to $^{19}$F and the outer coil tuned to H. The data were recorded without proton decoupling with a spectral width of 12000 Hz, an acquisition time of 0.8s and a relaxation delay of 2.8s. Chemical shifts are referenced to trifluoroacetic acid.

$IC_{50}$ determination was achieved by measuring the substrate signal as a function of the inhibitor concentration using one of the formulas below.

$$[S_w] = \frac{[S_{w/o}] - [S_{TOT}]}{1 + \left(\frac{[I]}{IC_{50}}\right)^n} + [S_{TOT}]$$

where $[S_w]$ and $[S_{w/o}]$ are given by the integrals of the substrate signal in the presence and absence of the inhibitor, respectively. $[S_{TOT}]$ is the substrate concentration used for the experiments and it represents an internal reference, [I] is the concentration of the inhibitor, $IC_{50}$ the concentration of the inhibitor at which 50% inhibition is observed and $n$ is the cooperativity factor (known also as Hill slope). In the absence of allosteric effects (i.e. $n = 1$) a meaningful value for $IC_{50}$ is derived with a single (or very few 2-3) experimental point. This is possible because the values for both plateaus are known. These are Sw/o for the 0% inhibition and $S_{TOT}$ for the 100% inhibition. $IC_{50}$ data were analyzed with non-linear least squares methods in the Origin 5.0 software package. Biochemical and cell-based assay data for representative compounds are reported in Table 1.

Table 1.

| Cpd. | AKT1 $IC_{50}$ (μM) Biochem assay | AKT2 $IC_{50}$ (μM) Biochem assay | AKT3 $IC_{50}$ (μM) Biochem assay | AKT1 $IC_{50}$ (μM) FABS NMR assay | PKAα $IC_{50}$ (μM) FABS NMR assay | S6 phosphor ylation (Ser235/236) in MCF7-cell $IC_{50}$ (μM) |
|---|---|---|---|---|---|---|
| 1 | 0.050 | 0.266 | 0.032 | 0.150 | 0.020 | 13.5 |
| 2 | 0.017 | 0.155 | 0.041 | 0.03 | 0.01 | |
| 3 | 0.184 | 1.988 | 0.187 | 0.760 | 0.075 | |
| 6 | 0.121 | 0.362 | 0.043 | 0.315 | 0.062 | 11.5 |
| 7 | 0.132 | 0.797 | 0.717 | | | |
| 8 | 0.431 | 1.859 | 1.064 | | | |

The compounds of the present invention can be administered either as single agents or, alternatively, in combination with known anticancer treatments such as radiation therapy or chemotherapy regimen in combination with cytostatic or cytotoxic agents, antibiotic-type agents, alkylating agents, antimetabolite agents, hormonal agents, immunological agents, interferon-type agents, cyclooxygenase inhibitors (e.g. COX-2 inhibitors), matrixmetalloprotease inhibitors, telomerase inhibitors, tyrosine kinase inhibitors, anti-growth factor receptor agents, anti-HER agents, anti-EGFR agents, anti-angiogenesis agents (e.g. angiogenesis inhibitors), farnesyl transferase inhibitors, ras-raf signal transduction pathway inhibitors, cell cycle inhibitors, other cdks inhibitors, tubulin binding agents, topoisomerase I inhibitors, topoisomerase II inhibitors, and the like.

If formulated as a fixed dose, such combination products employ the compounds of this invention within the dosage range described below and the other pharmaceutically active agent within the approved dosage range.

Compounds of formula (I) may be used sequentially with known anticancer agents when a combination formulation is inappropriate.

The compounds of formula (I) of the present invention, suitable for administration to a mammal, e.g., to humans, can be administered by the usual routes and the dosage level depends upon the age, weight, conditions of the patient and administration route.

For example, a suitable dosage adopted for oral administration of a compound of formula (I) may range from about 10 to about 500 mg per dose, from 1 to 5 times daily. The compounds of the invention can be administered in a variety of dosage forms, e.g., orally, in the form tablets, capsules, sugar or film coated tablets, liquid solutions or suspensions; rectally in the form suppositories; parenterally, e.g., intramuscularly, or through intravenous and/or intrathecal and/or intraspinal injection or infusion.

The present invention also includes pharmaceutical compositions comprising a compound of formula (I) or a pharmaceutically acceptable salt thereof in association with a pharmaceutically acceptable excipient, which may be a

carrier or a diluent.

The pharmaceutical compositions containing the compounds of the invention are usually prepared following conventional methods and are administered in a suitable pharmaceutical form. For example, the solid oral forms may contain, together with the active compound, diluents, e.g., lactose, dextrose saccharose, sucrose, cellulose, corn starch or potato starch; lubricants, e.g., silica, talc, stearic acid, magnesium or calcium stearate, and/or polyethylene glycols; binding agents, e.g., starches, arabic gum, gelatine methylcellulose, carboxymethylcellulose or polyvinyl pyrrolidone; disintegrating agents, e.g., starch, alginic acid, alginates or sodium starch glycolate; effervescing mixtures; dyestuffs; sweeteners; wetting agents such as lecithin, polysorbates, laurylsulphates; and, in general, non-toxic and pharmacologically inactive substances used in pharmaceutical formulations. These pharmaceutical preparations may be manufactured in known manner, for example, by means of mixing, granulating, tabletting, sugar-coating, or film-coating processes.

The liquid dispersions for oral administration may be, e.g., syrups, emulsions and suspensions. As an example, the syrups may contain, as carrier, saccharose or saccharose with glycerine and/or mannitol and sorbitol.

The suspensions and the emulsions may contain, as examples of carriers, natural gum, agar, sodium alginate, pectin, methylcellulose, carboxymethylcellulose, or polyvinyl alcohol. The suspension or solutions for intramuscular injections may contain, together with the active compound, a pharmaceutically acceptable carrier, e.g., sterile water, olive oil, ethyl oleate, glycols, e.g., propylene glycol and, if desired, a suitable amount of lidocaine hydrochloride.

The solutions for intravenous injections or infusions may contain, as a carrier, sterile water or preferably they may be in the form of sterile, aqueous, isotonic, saline solutions or they may contain propylene glycol as a carrier.

The suppositories may contain, together with the active compound, a pharmaceutically acceptable carrier, e.g., cocoa butter, polyethylene glycol, a polyoxyethylene sorbitan fatty acid ester surfactant or lecithin.

## EXPERIMENTAL SECTION

For a reference to any specific compound of formula (I) of the invention, optionally in the form of a pharmaceutically acceptable salt, see the experimental section and claims. Referring to the examples that follow, compounds of the present invention were synthesized using the methods described herein, or other methods, which are well known in the art.

The short forms and abbreviations used herein have the following meaning:

| | |
|---|---|
| g (grams) | mg (milligrams) |
| ml (milliliters) | mM (millimolar) |
| μM (micromolar) | mmol (millimoles) |
| h (hours) | MHz (Mega-Hertz) |
| mm (millimetres) | Hz (Hertz) |
| M (molar) | min (minutes) |
| mol (moles) | TLC (thin layer chromatography) |
| r.t. (room temperature) | TEA (triethylamine) |
| TFA (trifluoroacetic acid) | DMF (N,N-dimethyl formamide) |
| DIPEA (N,N-diisopropyl-N-ethylamine) | DCM (dichloromethane) |
| THF (tetrahydrofuran) | Hex (hexane) |
| MeOH (Methanol) | DMSO (dimethylsulfoxide) |
| TIPS (triisopropylsilyl) | bs (broad singlet) |
| TBDMS (dimethyl-tert-butylsilyl) | Ac (acetyl) |
| BOC (tert-butyloxycarbonyl) | $Ac_2O$ acetic anhydride |
| NaH = sodium hydride, 60% in mineral oil | ESI = electrospray ionization |

TBTU (2-(1H-benzotriazol-1-yl)-1,1,3,3-tetramethyluronium tetrafluoroborate RP-HPLC (reverse phase high performance liquid chromatography)

With the aim to better illustrate the present invention, without posing any limitation to it, the following examples are now given.

As used herein the symbols and conventions used in the processes, schemes and examples are consistent with those used in the contemporary scientific literature, for example, the *Journal of the American Chemical Society* or the *Journal of Biological Chemistry.*

Unless otherwise noted, all materials were obtained from commercial suppliers, of the best grade and used without further purification. Anhydrous solvent such as DMF, THF, $CH_2Cl_2$ and toluene were obtained from the Aldrich

Chemical Company. All reactions involving air- or moisture-sensitive compounds were performed under nitrogen or argon atmosphere.

**General purification and analytical methods**

Flash chromatography was performed using Aldrich Chemical Company silica gel (200-400 mesh, 60A).

HPLC was performed on Waters X Terra RP 18 (4,6 x 50 mm, 3.5 μm) column using a Waters 2790 HPLC system equipped with a 996 Waters PDA detector and Micromass mod. ZQ single quadrupole mass spectrometer, equipped with an electrospray (ESI) ion source. Mobile phase A was ammonium acetate 5 mM buffer (pH 5.5 with acetic acid-acetonitrile 95:5), and Mobile phase B was water-acetonitrile (5:95). Gradient from 10 to 90% B in 8 minutes, hold 90% B 2 minutes. UV detection at 220 nm and 254 nm. Flow rate 1 mL/min. Injection volume 10 microL. Full scan, mass range from 100 to 800 amu. Capillary voltage was 2.5 KV; source temperature was 120°C; cone was 10 V. Retention times (HPLC r.t.) are given in minutes at 220 nm or at 254 nm. Mass are given as m/z ratio.

When necessary, compounds were purified by preparative HPLC on a Waters Symmetry C18 (19 x 50 mm, 5 um) column or on a Waters X Terra RP 18 (30 x 150 mm, 5 μm) column using a Waters preparative HPLC 600 equipped with a 996 Waters PDA detector and a Micromass mod. ZMD single quadrupole mass spectrometer, electron spray ionization, positive mode. Mobile phase A was water-0.01% trifluoroacetic acid, and mobile phase B was acetonitrile. Gradient from 10 to 90% B in 8 min, hold 90% B 2 min. Flow rate 20 mL/min. In alternative, mobile phase A was water-0.1% $NH_3$, and mobile phase B was acetonitrile. Gradient from 10 to 100% B in 8 min, hold 100% B 2 min. Flow rate 20 mL/min.

1H-NMR spectrometry was performed on a Mercury VX 400 operating at 400.45 MHz equipped with a 5 mm double resonance probe [1H (15N-31P) ID_PFG Varian]. $^1$H-NMR were recorded in DMSO-d6 at 400 MHz, chemical shifts are expressed in ppm (δ).

The physical data given for the compounds exemplified is consistent with the assigned structure of those compounds.

**Example 1**

**Preparation of *N*-[(1*S*)-2-amino-1-benzylethyl]-6,7,8,9-tetrahydro-2*H*-2,3,6-triazabenzo[*cd*]azulene-8-carboxamide dihydrochloride [(Ia), (A= -$CH_2$-, X =-N(R6)$CH_2$-, Y= -CH=, R2= H, R1 = (1*S*)-2-amino-1-benzylethyl), R6=H]**

**Step 1. 1-(4-iodo-1*H*-pyrrolo[2,3-*b*]pyridin-3-yl)-*N,N*-dimethylmethanamine [(III), (R11 = R12 = Me)]**

730 mg (2.99 mmol) of 4-iodo-1*H*-pyrrolo[2,3-*b*]pyridine (X) were dissolved in 12 ml of n-butanol 264 mg (3.2 mmol) of dimethylamine hydrochloride and 100 mg (3.3 mmol) of paraformaldehyde were then added. The mixture was refluxed under stirring for 45 minutes. After that time the solvent was removed, the residue re-dissolved with water, basified with solid potassium carbonate and the product extracted with diethylether. The organic layer was finally dried over anhydrous sodium sulfate and the solvent evaporated. The crude was purified by chromatography on a silica gel column (eluant: $CH_2Cl_2$-$CH_3OH$-$NH_4OH$ 95-5-1) affording the title compound (420 mg 46 % yield) as yellowish solid.
1H NMR (400 MHz, DMSO-$d_6$) δ ppm 11.74 (s, 1H), 7.81 (d, *J*=5 Hz, 1H); 7.55 (d, *J*=5 Hz, 1H); 7.47 (m, 1H); 3.60 (bs, 1H); 2.21 (s, 1H).
MS ESI (M+H) calc. 302.0149; found 302.0150 ($C_{10}H_{12}IN_3$)

**Step 2. diethyl (acetamidomethyl)[(4-iodo-1*H*-pyrrolo[2,3-*b*]pyridin-3-yl)methyl]malonate [(V), (R7 = Methyl, R8= Ethyl)]**

1.95 g (6.48 mmol) of 1-(4-iodo-1*H*-pyrrolo[2,3-*b*]pyridin-3-yl)-*N,N*-dimethylmethanamine were dissolved in 45 ml of xylenes and 2 g (8.65 mmol) of diethyl (acetamidomethyl)malonate and 70 mg (1.75 mmol) of sodium hydrate were added. The resulting solution was refluxed under stirring for 2 hours. Ethylacetate was then added to the mixture. After washing with brine the organic layer was separated, dried over anhydrous sodium sulfate and evaporated. The crude was purified by chromatography on a silica gel column (eluant: $CH_2Cl_2$-$CH_3OH$ 95-5) affording the title compound (2.2 g 70 % yield) as light tan solid.
1H NMR (400 MHz, DMSO-$d_6$) δ ppm 11.74 (s, 1H); 7.80 (d, *J*=5 Hz, 1H); 7.55 (d, *J*=5 Hz, 1H); 7.47 (m, 1H); 3.98 (m, 2H); 3.72 (d, J=6.46 Hz, 2H); 3.64 (s, 2H); 1.83 (s, 3H); 1.03 (t, J=7.07 Hz, 3H);
MS ESI (M+H) calc. 488.0677; found 488.0697 ($C_{18}H_{22}IN_3O_5$)

**Step 3. 3-amino-2-[(4-iodo-1*H*-pyrrolo[2,3-*b*]pyridin-3-yl)methyl]propanoic acid [(VI)]**

220 mg (0.45 mmol) of diethyl (acetamidomethyl)[(4-iodo-1*H*-pyrrolo[2,3-*b*]pyridin-3-yl)methyl]malonate were dissolved in 5 ml of 37% hydrochloric acid and maintained at reflux for 6 hours. The solvent was then removed under reduced pressure and the residue re-dissolved in toluene twice and evaporated again to dryness.

The crude was finally triturated with ethanol , precipitated with diethylether and filtered, to give the title compound (150 mg 96 % yield) as an off-white solid.
1H NMR (400 MHz, DMSO-$d_6$) δ ppm 7.83 (d, *J*=4.88 Hz, 1 H); 7.56 (d, *J*=4.88 Hz, 1 H); 7.47 (d, *J*=2.44 Hz, 1 H) 3.09 (m, 5H).
MS ESI (M+H) calc. 346.0047; found 346.0054 ($C_{11}H_{12}N_3O_2$.HCl)

**Step 4. ethyl 3-amino-2-[(4-iodo-1*H*-pyrrolo[2,3-*b*]pyridin-3-1)methyl]propanoate [(VII), (R8= Ethyl)]**

To 150 mg (0.43 mmol) of 3-amino-2-[(4-iodo-1*H*-pyrrolo[2,3-*b*]pyridin-3-yl)methyl]propanoic acid and 25 mg of p-toluen-sulfonic acid, 15 ml of a mixture 1/1 of absolute ethanol and toluene were added. The reaction was maintained at reflux with a Dean Stark for 6 hours. The solvent was then removed in vacuo and the crude chromatographed on a silica gel column eluting with $CH_2Cl_2$-$CH_3OH$-$NH_4OH$ 95-5-1, affording the title compound (140 mg 86 % yield) as yellowish solid 1H NMR (400 MHz, DMSO-$d_6$) δ ppm 11.86 (s, 1H); 7.86 (m, 3H); 7.84 (d, *J*=5.00 Hz, 1H); 7.56 (d, *J*=5.00 Hz, 1H); 7.44 (d, *J*=2.32 Hz, 1H); 4.10 (q, *J*=7.00 Hz, 2H); 3.30 (m, 5H); 1.12 (d, *J*=7.00 Hz, 3H);
MS ESI (M+H) calc. 374.0360; found 374.0366 ($C_{13}H_{16}IN_3O_2$.Cl)

**Step 5. ethyl 6,7,8,9-tetrahydro-2*H*-2,3,6-triazabenzo[*cd*]azulene-8-carboxylate [(VIII), (R8= Ethyl)]**

125 mg (0.34 mmol) of ethyl 3-amino-2-[(4-iodo-1*H*-pyrrolo[2,3-*b*]pyridin-3-yl)methyl]propanoate were dissolved in melted diphenylether. The solution was maintained at 180°C under stirring for 1 hour. After this time the mixture was cooled to room temperature, re-dissolved in CH2Cl2/CH3OH 95/5 and charged on a silica gel column. Diphenylether was removed by eluting with dichloromethane and the product by eluting with dichloromethane-methanol 9/1. The title compound (54 mg 65 % yield) was obtained as off-white solid
1H NMR (500 MHz, DMSO-$d_6$) δ ppm 7.70 (d, *J*=5.61 Hz, 1H); 6.99 (br. s., 1H); 6.19 (d, *J*=5.85 Hz, 1H); 4.09 (q, *J*=7.07 Hz, 2 H); 3.70 (dd, *J*=13.78, 5.00 Hz, 1H); 3.18 (dd, *J*=15.37, 2.68 Hz, 1H); 2.93 (dd, *J*=15.00, 10.00 Hz, 1H); 2.86 (t, *J*=9.15 Hz, 1H) 1.19 (t, *J*=7.20 Hz, 3H).
MS ESI (M+H) m/z 246.

**Step 6. 6,7,8,9-tetrahydro-2*H*-2,3,6-triazabenzo[*cd*]azulene-8-carboxylic acid [(IXa), (A= -$CH_2$-, X = -N(R6)$CH_2$-, Y= -CH=, R2=H, R6=H)]**

125 mg (0.5 mmol) of ethyl 6,7,8,9-tetrahydro-2*H*-2,3,6-triazabenzo[*cd*]azulene-8-carboxylate were dissolved in 1 mL of ethanol and 1 mL of 1N sodium hydrate was added. The mixture was maintained at room temperature under stirring for 2 hours. Ethanol was removed under reduced pressure, water and 1 N hydrochloric acid were added up to neutralization and the resulting mixture driven to dryness. The residue was triturated with diethylether affording, after filtration, the title compound (100 mg 93 % yield) as off-white solid.
1H NMR (500 MHz, DMSO-$d_6$) δ ppm 10.90 (s, 1H); 7.65 (d, *J*=5.42 Hz, 1H); 7.44 (bs, 1H); 6.96 (BS, 1H); 6.90 (bs, 1H); 6.88 (bs, 1H); 6.10 (d, *J*=5.42 Hz, 1H); 3.57 (dd, *J*=5.85, 13.90 Hz, 1H); 3.19 (m, 1H); 3.07 (m, 1H); 2.85 (m, 1H); 2.60 (m, 1H);
MS ESI (M+H) calc. 218.1074; found 218.1064 ($C_{11}H_{11}N_3O_2$)

**Step 7. *tert*-butyl {(2*S*)-3-phenyl-2-[(6,7,8,9-tetrahydro-2*H*-2,3,6-triazabenzo[*cd*]azulen-8-ylcarbonyl)amino]propyl}carbamate [(Ia), (A= -$CH_2$-, X = -N(R6)$CH_2$-, Y= -CH=, R2=H, R3 = benzyl, R4=H, R5 = *tert*-butyl carbamate, R6=H)]**

100 mg (0.46 mmol) of 6,7,8,9-tetrahydro-2*H*-2,3,6-triazabenzo[*cd*]azulene-8-carboxylic acid were dissolved in 10 mL of dry dichloromethane and 5 mL of dimethylformamide. 60 μL (O.34 mmol) of DIPEA, 170 mg (0.5 mmol) of hydroxybenzotriazole and 170 mg (0.5 mmol) of TBTU were added consecutively. After 30 minutes under stirring at room temperature 50 mg (0.5 mmol) of *tert*-butyl [(2*S*)-2-amino-3-phenylpropyl]carbamate were added and the reaction mixture stirred overnight. The solvent was then evaporated in vacuo and the residue chromatographed on a silica gel column (eluent: $CH_2Cl_2$-MeOH 95/5) affording the title compound (100 mg 49 % yield) as pale yellow solid.

**Step 8.** ***N*-[(1*S*)-2-amino-1-benzylethyl]-6,7,8,9-tetrahydro-2*H*-2,3,6-triazabenzo[*cd*]azulene-8-carboxamide dihydrochloride [(Ia), (A= -$CH_2$-, X =-N(R6)$CH_2$-, Y= -CH=, R2= H, R3 = benzyl, R4= R5= H, R6=H)]**

**Cpd. 4**

60 mg (0.13 mmol) of *tert*-butyl {(2*S*)-3-phenyl-2-[(6,7,8,9-tetrahydro-2*H*-2,3,6-triazabenzo[*cd*]azulen-8-ylcarbonyl)amino]propyl}carbamate (Ia) were dissolved with 4 mL of dioxane and 2 mL of 4M HCl in dioxane were added. After 4 hours the mixture was concentrated to small volume, diethylether was added to precipitate the product that was collected by filtration, affording the title compound (45 mg 82 % yield) as off-white solid.

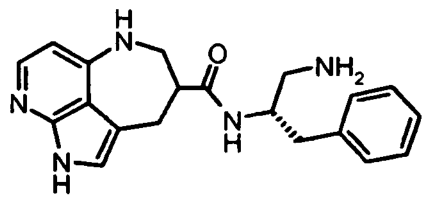

1H NMR (500 MHz, DMSO-$d_6$) δ ppm 13.43 (bs, 3H); 12.05 (d, *J*=6.58 Hz, 1H); 8.77 (d, *J*=18.78 Hz, 1H); 7.79 (m, 1H); 7.30 (m, 5H); 7.06 (d, *J*=16.10 Hz, 1H); 6.47 (d, *J*=9.27 Hz, 1H); 4.19 (m, 1H); 3.13 (d, *J*=15.73 Hz, 2H);
MS ESI (M+H) calc. 350.1975; found 350.1989 ($C_{20}H_{23}N_5O.2HCl$)
Operating in an analogues way, the following compound was prepared:

**6,7,8,9-tetrahydro-2*H*-2,3,6-triazabenzo[*cd*]azulene-8-carboxamide [(Ia),** (A=-**$CH_2$-, X = -N(R6)$CH_2$-, Y= -CH=, R2=H, R1 = H, R6=H)$_1$**

**Cpd. 5**

217 mg (1 mmol) of 6,7,8,9-tetrahydro-2*H*-2,3,6-triazabenzo[*cd*]azulene-8-carboxylic acid were dissolved 10 mL of tetrahydrofuran and 10 mL of dimethylformamide. 172 μL (2 mmol) of N,N-diisopropyl-N'-ethylamine , 191 mg (1 mmol) of EDCI, 150 mg (1 mmol) of N-hydroxy benzotriazole ammonium salt were added consecutively. The mixture was stirred at room temperature overnight. The solvent was then removed in vacuo, the residue re-dissolved with dichloromethane and washed with water. The organic layer was dried over sodium sulfate and evaporated. Trituration with diisopropylether gave the title compound (180 mg 83 % yield) as a white solid.

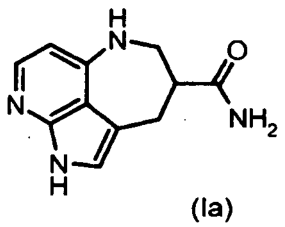

(Ia)

1H NMR (500 MHz, DMSO-$d_6$) δ ppm 10.91 (s, 1H); 7.68 (d, *J*=5.42 Hz, 1H); 7.42 (bs, 1H); 6.92 (BS, 1H); 6.88 (bs, 1H); 6.86 (bs, 1H); 6.13 (d, *J*=5.42 Hz, 1H); 3.55 (dd, *J*=5.85, 13.90 Hz, 1H); 3.19 (m, 1H); 3.08 (m, 1H); 2.85 (m, 1H); 2.61 (m, 1H);
MS ESI (M+H) calc. 217.1084; found 217.1074 ($C_{11}H_{12}N_4O$)

## Example 2

**Preparation of 6-acetyl-*N*-[(1*S*)-2-amino-1-benzylethyl]-6,7-dihydro-2*H*-2,3,6-triazabenzo[*cd*]azulene-8-carboxamide [(Ib), (A= -CH=, X= -N(R6)$CH_2$-, Y= -CH=, R6 = COR7, R7= Methyl, R1 =(1*S*)-2-amino-1-benzylethyl)]**

**Step 1.** **ethyl 2,6-diacetyl-6,7,8,9-tetrahydro-2*H*-2,3,6-triazabenzo[*cd*]azulene-8-carboxylate [(XII), (A= -$CH_2$-, X= -N(R6)$CH_2$-, Y= -CH=, R2 = R6 = -COR7, R7= Methyl, R8 = Ethyl)]**

100 mg (0.41 mmol) of ethyl 6,7,8,9-tetrahydro-2*H*-2,3,6-triazabenzo[*cd*]azulene-8-carboxylate (VIII) were refluxed in 4 mL of acetic anhydride under stirring for 2 hours. The solvent was then removed under reduced pressure and the crude chromatographed on a silica gel column by eluting with CH2C12-CH2COCH3 95/5, to give the title compound (72 mg 50 % yield) of an off-white solid.

**Step 2. ethyl 2,6-diacetyl-6,7-dihydro-2*H*-2,3,6-triazabenzo[*cd*]azulene-8-carboxylate [(XIII), (A= -CH=, X= -N(R6) $CH_{2}$-, Y= -CH=, R2 = R6 = -COR7, R7= Methyl, R8 = Ethyl)]**

100 mg (0.3 mmol) of ethyl 2,6-diacetyl-6,7,8,9-tetrahydro-2*H*-2,3,6-triazabenzo[*cd*]azulene-8-carboxylate were dissolved in 16 mL of dichloromethane. 100 mg (0.44 mmol) of 2,3-dichloro-5,6-dicyano-p-benzoquinone (DDQ) were added and the mixture stirred at room temperature, under argon atmosphere overnight. The reaction mixture was then diluted by dichloromethane and filtered through a celite pad. The reddish filtrate was washed with a saturated solution of sodium carbonate until to make colorless, dried over anhydrous sodium sulfate and evaporated in vacuo. The crude was purified by chromatography on a silica gel column (eluent: CH2Cl2-CH3COCH3 95/5) to obtain the title compound (60 mg 61 % yield) of yellowish solid.
1H NMR (500 MHz, DMSO-$d_6$) δ ppm 8.46 (s, 1H), 8.45 (d, J= 5.49 Hz, 1H), 7.96 (bs, 1H), 7.47 (d, J= 5.49 Hz, 1H), 5.63 (b, 2H), 4.24 (q, *J*=7.02 Hz, 2H), 3.00 (s, 3 H), 2.20 (br. s., 3 H) 1.29 (t, *J*=7.10 Hz, 3 H).

**Step 3. 6-acetyl-6,7-dihydro-2*H*-2,3,6-triazabenzo[*cd*]azulene-8-carboxylic** acid **[(IX), (A= -CH=, X= -N(R6)$CH_2$-, Y= -CH=, R6 = -COR7, R7= Methyl)]**

90 mg (0.28 mmol) of ethyl 2,6-diacetyl-6,7-dihydro-2*H*-2,3,6-triazabenzo[*cd*]azulene-8-carboxylate were dissolved in 5 mL of ethanol and 5 mL of 1 N sodium hydrate were added. The mixture was stirred at room temperature for 60 hours. After that time ethanol was removed in vacuo, the residue was re-dissolved with water, extracted twice with dichloromethane, neutralized with 2N HCl. The resulting precipitate was collected by filtration, washed with water and exsiccated under reduced pressure. The product was chromatographed on a silica gel column (eluent: CH2Cl2-MeOH-AcOH-H2O 80/20/7/3) to give the title compound (65 mg 89 % yield) of a off-white solid.
1H NMR (500 MHz, DMSO-$d_6$) δ ppm 12.47 (bs, 1H), 8.25 (d, J= 5.24 Hz, 1H), 7.99 (m, 2H), 7.24 (d, J= 5.24 Hz, 1H), 5.77 (b, 2H), 4.24 (q, *J*=7.10 Hz, 2H), 2.20 (br. s., 3 H) 1.30 (t, *J*=7.10 Hz, 3 H).

**Step 4. *tert*-butyl [(2*S*)-2-{[(6-acetyl-6,7-dihydro-2*H*-2,3,6-triazabenzo[*cd*]azulen-8-yl)carbonyl]amino}-3-phenylpropyl]carbamate [(Ib), (A= -CH=, X= -N(R6)$CH_2$, Y= -CH=, R6 = -COR7, R7= Methyl, R3 = benzyl, R4 =H, R5 = *tert*-butyl-carbamate)]**

65 mg (0.25 mmol) of 6-acetyl-6,7-dihydro-2*H*-2,3,6-triazabenzo[*cd*]azulene-8-carboxylic acid were dissolved in 6 mL of dry dimethylformamide and 3 mL of dichloromethane under argon atmosphere. 130 μl (0.75 mmol) of DIPEA, 72 mg (0.37 mmol) of EDCl, 57 mg (0.37 mmol) of HOBT and 63 mg (0.25 mmol) of *tert*-butyl [(2*S*)-2-amino-3-phenylpropyl]carbamate were added consecutively. The reaction mixture was stirred at room temperature overnight. The solvent was then evaporated in vacuo The residue was re-dissolved with dichloromethane and washed with a saturated aqueous solution of NaHC03. The organic layer was finally dried over sodium sulfate and evaporated. The product was chromatographed on a silica gel column (eluent: CH2C12-CH3OH 95/5) to afford the title compound (100 mg 82 % yield) of a pale yellow solid.

**Step 5. 6-acetyl-*N*-[(1*S*)-2-amino-1-benzylethyl]-6,7-dihydro-2*H*-2,3,6-triazabenzo[*cd*]azulene-8-carboxamide [(Ib), (A= -CH=, X= -N(R6)-$CH_2$-, Y=-CH=, R6 = -COR7, R7= Methyl, R1 = (1*S*)-2-amino-1-benzylethyl)]**

**Cpd. 3**

100 mg (0.20 mmol) of *tert*-butyl [(2*S*)-2-{[(6-acetyl-6,7-dihydro-2*H*-2,3,6-triazabenzo[*cd*]azulen-8-yl)carbonyl] amino}-3-phenylpropyl]carbamate were dissolved with 4 mL of dioxane and 1 mL of 4M HCl in dioxane was added. After 1 hour under stirring at room temperature the solvent was removed in vacuo and the residue triturated with diethylether and filtered. The precipitate was chromatographed on a silica gel column (eluent: CH2Cl2-MeOH-NH40H 30 % 95/5/1) to afford the title compound (60 mg), whichwas converted into the corresponding hydrochlorides by treatment with 1 mL of 4M HCl in dioxane and evaporated in vacuo, and the residue triturated with diethyl ether and filtered.

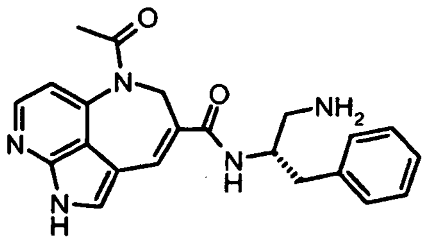

1H NMR (400 MHz, DMSO-$d_6$) δ ppm, 12.40 (s, 1H). 8.23 (d, *J*=5.49 Hz, 1 H), 8.21 (d, *J*=8.66 Hz, 1 H) 7.92 (bs, 3H), 7.83 (d, *J*=2.44 Hz, 1 H), 7.60 (s, 1H), 7.26 (m, 6H), 4.30 (m, 1H), 2.87 (m, 4H), 2.18 (s, 3H)
MS ESI (M+H) calc. 390.1924; found 390.1931 ($C_{22}H_{23}N_3O_2$ .HCl))

**Example 3**

**Preparation of *N*-{(1*S*)-2-amino-1-benzylethyl]-6,7-dihydro-2*H*-2,3,6-triazabenzo[*cd*]azulene-8-carboxamide hydrochloride. [(Ib), (A= -CH=, X=-N(R6)$CH_{2-}$, Y= -CH=, R2= R6 = H, R1 = (1*S*)-2-amino-1-benzylethyl)]**

**Step 1. 6,7-dihydro-2*H*-2,3,6-triazabenzo[*cd*]azulene-8-carboxylic acid [(IXb), (A= -CH=, X= -N(R6)$CH_{2-}$, Y= -CH=, R2= R6 = H)]**

65 mg (0.25 mmol) of 6-acetyl-6,7-dihydro-2*H*-2,3,6-triazabenzo[*cd*]azulene-8-carboxylic acid (IXb) were dissolved in a mixture of 4 mL of dioxane and 1 ml of water. To this solution 1 mL of 4M HCl in dioxane was added. After 18 hour under stirring at room temperature the solvent was removed in vacuo and the residue triturated with diethylether and filtered, to obtain the title compound as hydrochloride salt (63 mg 100% yield).

**Step 2. *tert*-butyl [(2*S*)-2-{[(6,7-dihydro-2*H*-2,3,6-triazabenzo[*cd*]azulen-8-yl)carbonyl]amino}-3-phenylpropyl] carbamate [(Ib), (A= -CH=, X= -N(R6)$CH_2$-, Y= -CH=, R2= R6 = H, R3 = benzyl, R4 =H, R5 = *tert*-butyl-carbamate)]**

63 mg (0.25 mmol) of 6,7-dihydro-2*H*-2,3,6-triazabenzo[*cd*]azulene-8-carboxylic acid were dissolved in 6 mL of dry dimethylformamide and 3 mL of dichloromethane under argon atmosphere. 130 μl (0.75 mmol) of DIPEA, 72 mg (0.37 mmol) of EDCI, 57 mg (0.37 mmol) of HOBt and 63 mg (0.25 mmol) of *tert*-butyl [(2*S*)-2-amino-3-phenylpropyl] carbamate were added consecutively. The reaction mixture was stirred at room temperature overnight. The solvent was then evaporated in vacuo The residue was re-dissolved with dichloromethane and washed with a saturated aqueous solution of NaHCO3. The organic layer was finally dried over sodium sulfate and evaporated. The product was chromatographed on a silica gel column (eluent: CH2Cl2-CH3OH 95/5) to afford the title compound (52 mg 45 % yield) of a pale yellow solid.

**Step3. *N*-[(1*S*)-2-amino-1-benzylethyl]-6,7-dihydro-2*H*-2,3,6-triazabenzo[*cd*]azulene-8-carboxamide hydrochloride [(Ib), (A= -CH=, X=-N(R6)$CH_2$-, Y= -CH=, R2= R6 = H, R1 = (1*S*)-2-amino-1-benzylethyl)]**

**Cpd 2**

52 mg (0.11 mmol) of *tert*-butyl [(2*S*)-2-{[(6,7-dihydro-2*H*-2,3,6-triazabenzo[*cd*]azulen-8-yl)carbonyl]amino}-3-phenylpropyl]carbamate were dissolved with 4 mL of dioxane and 1 mL of 4M HCl in dioxane was added. After 1 hour under stirring at room temperature the solvent was removed in vacuo and the residue triturated with diethylether and filtered to give the title compound (35 mg 83% yield) of a white solid.

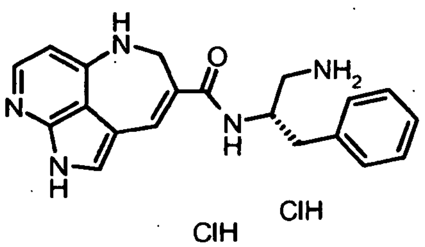

1H NMR (400 MHz, DMSO-$d_6$) δ ppm 12.72 (s, 1H), 8.62 (bs, 1H), 8.29 (d, *J*=8.41 Hz, 1 H), 7.96 (bs, 1H), 7.92 (d, *J*=6.83 Hz, 1 H), 7.50 (m, 2H), 7.27 (m, 5H), 6.56 (d, *J*=6.83 Hz, 1 H), 4.30 (tq, *J*=7.00 Hz, 1 H), 4.24 (dd, *J*=15.50, 3.80 Hz, 1 H), 4.15 - 4.20 (m, *J*=15.50, 3.60 Hz, 1 H), 2.94 (dt, *J*=11.86, 6.08 Hz, 1 H), 2.86 (d, *J*=7.19 Hz, 1 H).
MS ESI (M+H) calc. 348.1819; found 348.1823 ($C_{20}H_{21}$N5O.2HCl))

**Example 4**

**Preparation of *N*-[(1*S*)-2-amino-1-benzylethyl]-6,7-dihydro-2*H*-2,3-diacabenco[*cd*]azulene-8-carboxamide dihydrochloride [(Ic), (A= -CH=, X= -$CH_2$-$CH_2$-, Y= -CH=, R2= H, R1 = (1*S*)-2-amino-1-benzylethyl)]**

**Step 1. 4-iodo-1-(triisopropylsilyl)-1*H*-pyrrolo[2,3-*b*]pyridine, [(XX) (R9= TIPS)]**

1.8 g (7.4 mmol) of 4-iodo-1*H*-pyrrolo[2,3-*b*]pyridine (X) were dissolved with 30 mL of dry tetrahydrofuran. The resulting solution was cooled to 0°C and 8.2 ml of 1N solution in THF of sodium bis(trimethylsilyl)amide were added under stirring and in argon atmosphere. After 15 minutes in the same conditions 2 mL of trisopropylsilyl chloride (9.3 mmol) were added dropwise. The reaction mixture was stirred at 0°C for 30 minutes and further 30 minutes at room temperature. The solvent was then removed in vacuo, the residue taken up with dichloromethane and icy water and the layers separated. The organic phase was dried over sodium sulfate and evaporated to dryness. The crude was chromatographed on a silica gel column (eluent: n-hexane) to afford the title compound (1.7 g 57 % yield) as an yellowish oil.

**Step 2. methyl (3*E*)-4-[1-(triisopropylsilyl)-1*H*-pyrrolo[2,3-*b*]pyridin-4-yl]but-3-enoate [(XXI), (R8= Methyl, and R9 = TIPS, n=1)]**

1.3 g (3.3 mmol) of 4-iodo-1-(triisopropylsilyl)-1*H*-pyrrolo[2,3-*b*]pyridine were dissolved in 20 mL of dry acetonitrile and 1.34 mL of triethylamine, 50 mg of palladium acetate, 100 mg of tris(o-tolyl)phosphine and 1.05 mL (9.9 mmol) of methyl 3-butenoate were added consecutively. under argon atmosphere. The reaction mixture was heated at 100°C under stirring for 5 hours. The solvent was then evaporated and the crude chromatographed on a silica gel column by eluting with dicloromethane-methanol 95/5, to give the title 0.55 g (45 % yield) of a mixture of methyl (3*E*)-4-[1-(triisopropylsilyl)-1*H*-pyrrolo[2,3-*b*]pyridin-4-yl]but-3-enoate (XXIa) and methyl (2*E*)-4-[1-(triisopropylsilyl)-1*H*-pyrrolo[2,3-*b*]pyridin-4-yl]but-2-enoate (XXIb).
(XXIa) and (XXIb) mixture:

1H NMR (400 MHz, DMSO-$d_6$) δ ppm 8.20 and 8.22 (2d, *J*= 4.98 Hz, 1H), 7.29 and 7.33 (2d, *J*=3.81 Hz, 1H); 7.08 (d, *J*=4.98 Hz, 1H); 6.79 (m, 1H); 6.72 (d, *J*=3.52 Hz, 1H); 6.66 (m, 1H); 3.72 and 3.74 (2s, 3H); 3.36 (dd, *J*=1.17, 7.03 Hz, 2H); 1.84 (m, 3H); 1.13 (d, *J*=7.62 Hz, 18H).

**Step 3. methyl 4-[1-(triisopropylsilyl)-1*H*-pyrrolo[2,3-*b*]pyridin-4-yl]butanoate [(XXII), (R8= Methyl, R9 = TIPS, n=1)]**

0.55 g (1.48 mmol) of the previous obtained mixture were dissolved with 25 mL of absolute ethanol and 50 mg of palladium on charcoal 5 % were added. The mixture was hydrogenated in a Parr apparatus at a pressure of 30 psi for 5 hours. After filtration through a celite pad the filtrate was evaporated in vacuo to give the title compound (0.4 g 72% yield) as an yellowish oil.
1H NMR (400 MHz, DMSO-$d_6$) δ ppm 8.17 (d, *J*=4.85 Hz, 1H) 7.26 (d, *J*=4.00 Hz, 1H); 6.83 (d, 1H); 6.58 (d, *J*=4.00 Hz, 1H); 3.67 (s, 3H); 2.91 (t, *J*=7.33 Hz, 2H); 2.39 (t, *J*=7.33 Hz, 2H); 2.10 (m, 2H); 1.86 (m, 3H); 1.13 (d, *J*=7.62 Hz, 18 H).

**Step 4. methyl 4-(1*H*-pyrrolo[2,3-*b*]pyridin-4-yl)butanoate [(XXIII), (R8= Methyl, n=1)]**

400 mg (1.07 mmol) of methyl 4-[1-(triisopropylsilyl)-1*H*-pyrrolo[2,3-*b*]pyridin-4-yl]butanoate were dissolved in 2 mL of dry tetrahydrofuran and 2 mL of tetrabutyl ammonium fluoride (TBAF) 1 N in THF were added under argon atmosphere. The reaction mixture was stirred at room temperature for 30 minutes and then diluted with dichloromethane. 40 mL of an aqueous saturated sodium carbonate solution were added, the phases separated and the organic layer dried over sodium sulfate and evaporated. The crude was chromatographed on a silica gel column (eluent: CH2Cl2-CH3COCH3 4/1) to give the title compound (200 mg 87 % yield) as a white solid.
1H NMR (400 MHz, DMSO-$d_6$) δ ppm 9.40 (bs, 1H), 8.20 (d, *J*=5.00 Hz, 1H), 7.31 (d, *J*=3.68 Hz, 1H), 6.94 (d, *J*=5.00 Hz, 1H), 6.58 (d, *J*=3.68 Hz, 1H), 3.67 (s, 3H); 2.97 (t, *J*=7.03 Hz, 2H), 2.38 (t, *J*=7.33 Hz, 2H), 2.10 (m, 1H).

**Step 5. methyl 4-(3-formyl-1*H*-pyrrolo[2,3-*b*]pyridin-4-yl)butanoate [(XXIV), (R8= Methyl, n=1)]**

190 mg (0.87 mmol) of 4-(1*H*-pyrrolo[2,3-*b*]pyridin-4-yl)butanoate were dissolved with 4 mL of nitromethane and 4 mL of dichloromethane under argon atmosphere. The resulting solution was cooled to 0°C and 350 mg (2.6 mmol) of aluminum trichloride and 162 μL of dichloromethyl methyl ether (1.8 mmol) were added consecutively. The reaction mixture was stirred for 7 hours , then poured into icy water and extracted several times with ethylacetate. The organic layer was dried over sodium sulfate and evaporated in vacuo. The crude was chromatographed on a silica gel column (eluent: dichloromethane-methanol 95/5) to give the title compound (150 mg 70 % yield) as a white solid.
1H NMR (400 MHz, DMSO-$d_6$) δ ppm; 9.94 (s, 1H), 8.33 (d, *J*=4.88 Hz, 1H); 8.06 (s, 1H); 7.14 (d, *J*=4.88 Hz, 1H); 3.66 (s, 3H); 3.38 (t, *J*=7.62 Hz, 2H); 2.45 (t, *J*=7.33 Hz, 2H); 2.01 (m, 2H).

**Step 6. 4-(3-formyl-1*H*-pyrrolo[2,3-*b*]pyridin-4-yl)butanoic acid [(XXV), (n=1)]**

95 mg (0.40 mmol) of methyl 4-(3-formyl-1*H*-pyrrolo[2,3-*b*]pyridin-4-yl)butanoate were dissolved in 4 mL of methanol and 1 mL of 1 N sodium hydrate were added. The solution was stirred at room temperature for 6 hours. The solvent was removed, the residue re-dissolved with water and neutralized with 1N HCl. The solvent was evaporated and toluene was added several times and evaporated again. The crude was triturated with diethylether and the product collected by filtration, to obtain the title compound (90 mg 90%Yield) as an off-white solid.
1H NMR (400 MHz, DMSO-$d_6$) δ ppm 12.74 (bs, 1H), 11.97 (bs, 1H); 8.45 (s, 1H); 9.81 (s, 1H); 8.24 (d, *J*=4.72 Hz,1H); 7.06 (d, *J*=4.72 Hz, 1H); 3.22 (t, *J*=7.62 Hz, 2H); 2.23 (t, *J*=7.33 Hz, 2H); 1.78 (m, 2H)..

**Step 7. 2-acetyl-6,7-dihydro-2*H*-2,3-diazabenzo[*cd*]azulene-8-carboxylic acid [(XXVI), (n=2, R7 = Methyl)]**

To 90 mg (0.38 mmol) of 4-(3-formyl-1*H*-pyrrolo[2,3-*b*]pyridin-4-yl)butanoic acid 3 mL of acetic anhydride and 6 mL of triethylamine were added. The mixture was heated at 100°C under argon atmosphere for 24 hours. The reaction was cooled to room temperature, then 10 mL of water were added and the stirring maintained for 15 minutes. After that time the solvent was removed and the residue partitioned between n-butanol and water. The phases were then separated and the organic layer dried over sodium sulfate and evaporated to dryness. The resulting crude was chromatographed on a silica gel column by eluting with CH2Cl2-CH3COOH-MeOH 95/0.5/4.5 to give the title compound (57 mg 70 % yield) as light tan solid.
1H NMR (400 MHz, DMSO-$d_6$) δ ppm 12.37 (bs, 1H), 8.32 (d, *J*=4.98 Hz, 1H); 8.30 (s, 1H); 7.80 (s, 1H);7.18 (d, *J*=4.98 Hz, 1H); 3.07 and 2.88 (m, 4H); 2.98 (s, 3H).

**Step 8. 6,7-dihydro-2*H*-2,3-diazabenzo[*cd*]azulene-8-carboxylic acid [(IXc), (A= - CH=, X= -$CH_2$-$CH_2$-, Y= -CH=, R2=H)**

57 mg (0.22 mmol) of 2-acetyl-6,7-dihydro-2*H*-2,3-diazabenzo[*cd*]azulene-8-carboxylic acid were dissolved with 2 mL of methanol and 2 mL of 1N sodium hydrate were added under argon atmosphere. The mixture was stirred at room temperature for 3 hours. The methanol was then distilled off and the remaining solution neutralized with 1N hydrochloric acid and evaporated to dryness. The residue was partitioned between n-butanol and water. The organic layer was dried over sodium sulfate and evaporated. The crude was purified by chromatography on a silica gel column (eluent: CH2C12-MeOH 95/5) to afford the title compound (45 mg 100 % yield) as pale yellow solid.
1H NMR (400 MHz, DMSO-$d_6$) δ ppm 12.00 and 12.04 (2 bs, 2H), 8.14 (d, *J*=4.69 Hz, 1H); 7.83 (s, 1H);7.79 (s, 1H); 6.90 (d, *J*=4.69 Hz, 1H); 3.01 and 2.85 (2m, 4H).

**Step 9. *tert*-butyl {(2*S*)-2-[(6,7-dihydro-2*H*-2,3-diazabenzo[*cd*]azulen-8-ylcarbonyl)amino]-3-phenylpropyl}carbamate [(Ic), (A= -CH=, X= -$CH_2$-$CH_2$-, Y= -CH=, R1 = (1*S*)-2-amino-1-benzylethyl)]**

220 mg (1 mmol) of 6,7-dihydro-2*H*-2,3-diazabenzo[*cd*]azulene-8-carboxylic acid were dissolved in 40 mL of dry dichloromethane and 10 mL of dimethylformamide under argon atmosphere. 1 mL (4 mmol) of DIPEA, 250 mg (1.3 mmol) of EDCI, 153 mg (1 mmol) of HOBT were added consecutively. After 30 minutes under stirring at room temperature 250 mg of *tert*-butyl [(2*S*)-2-amino-3-phenylpropyl]carbamate were added and the solution stirred overnight. The solvent was then removed in vacuo, the residue partitioned between dichloromethane and aqueous sodium hydrogenocarbonate. The organic phase was finally dried over sodium sulfate and evaporated. The crude was chromatographed on a silica gel column (eluent: CH2Cl2-CH3OH-NH4OH 30 % 95/5/1) to furnish the title compound (250 mg 65 % yield) as pale yellow solid.

**Step 10.** ***N*-[(1*S*)-2-amino-1-benzylethyl]-6,7-dihydro-2*H*-2,3-diazabenzo[*cd*]azulene-8-carboxamide dihydrochloride [(Ic), (A= -CH=, X= -$CH_2$-$CH_2$-, Y= -CH=, R2= H, R1 = (1*S*)-2-amino-1-benzylethyl)]**

**Cpd. 1**

250 mg (0.72 mmol) of *tert*-butyl {(2*S*)-2-[(6,7-dihydro-2*H*-2,3-diazabenzo[*cd*]azulen-8-ylcarbonyl)amino]-3-phenylpropyl}carbamate were dissolved in 20 mL of dry dioxane and 8 mL of 4 N HCl in dioxane were added. After 2 hours under stirring at room temperature the solution was concentrated in vacuo, diethylether was added and the formed precipitate collected by filtration, to afford the title compound (270 mg 90 % yield) as off white solid.

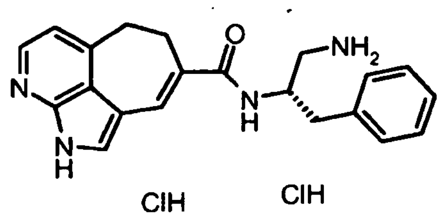

1H NMR (400 MHz, DMSO-$d_6$) δ ppm 8.19 (d, *J*=4.90 Hz, 1H); 12.14 (s, 1H), 8.01 (d, *J*=8.78 Hz, 1H); 7.92 (bs, 3H); 7.69 (d, *J*=2.56 Hz, 1H); 7.40 (s, 1H); 7.34 (m, 2H); 7.29 (m, 2H); 7.23 (m, 1H);6.98 (d, 1H, *J*=4.90 Hz, 1H); 4.31 (s, 1H); 2.7-3.1 (4m, 8H);.
MS ESI (M+H) calc. 347.1866; found 347.1869 ($C_{21}H_{22}N_4O.2HCl$))

According to the same methodology the following compound can be prepared:

***N*-[(1*S*)-1-phenyl-2-pyrrolidin-1-ylethyl]-6,7-dihydro-2*H*-2,3-diazabenzo[*cd*]azulene-8-carboxamide [(Ic), (A= -CH=, X= -$CH_2$-$CH_2$-, Y= -CH=, R2=H, R1= (1*S*)-1-phenyl-2-pyrrolidin-1-ylethyl)]**

**Cpd. 6**

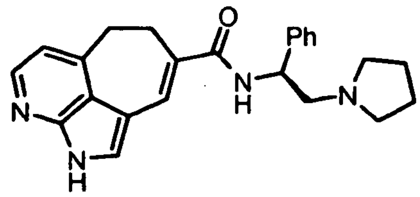

1H NMR (400 MHz, DMSO-$d_6$) δ ppm 11.94 (s, 1H), 8.29 (bs, 1H);8.14 (d, *J*=4.76 Hz, 1H); 7.69 (d, *J*=2.56 Hz, 1H); 7.49 (bs, 1H); 7.42 (m, 2H); 7.34 (m, 2H); 7.24 (m, 1H); 6.92 (d, *J*=4.76 Hz, 1H); 5.08 (m, 1H); 2.60-3.10 (several m, 8H).
MS ESI (M+H) calc. 387.2179; found 387.2184 ($C_{24}H_{26}N_4O$)

**Example 5**

**Preparation of *N*-[(1*S*)-2-amino-1-benzylethyl]-1,2-dihydropyrrolo[4,3,2-*ij*]isoquinoline-4-carboxamide dihydrochloride [(Id), (A= -CH=, X= -CH=, Y=-$CH_2$-, R2=H, R1 = (1*S*)-2-amino-1-benzylethyl)]**

**Step 1.** **1-[*tert*-butyl(dimethyl)silyl]-4-iodo-1*H*-pyrrolo[2,3-*b*]pyridine [(XX), (R9= TBDMS).**

1.8 g (7.4 mmol) of 4-iodo-1*H*-pyrrolo[2,3-*b*]pyridine (X) were dissolved with 30 mL of dry tetrahydrofuran. The resulting solution was cooled to 0°C and 8.2 mL of 1N solution in THF of sodium bis(trimethylsilyl)amide were added under stirring and in argon atmosphere. After 15 minutes in the same conditions 1.6 mL (9.3 mmol) of *tert* butyldimethylsilyl chloride were added dropwise. The reaction mixture was stirred at 0°C for 30 minutes and further 30 minutes at room temperature. The solvent was then removed in vacuo, the residue taken up with dichloromethane and icy water and the

layers separated. The organic phase was dried over sodium sulfate and evaporated to dryness. The crude was chromatographed on a silica gel column (eluent: n-hexane) to afford the title compound (1.51 g 57 % yield) as an yellowish oil.

**<u>Step 2.</u> ethyl (2*E*)-3-{1-[*tert*-butyl(dimethyl)silyl]-1*H*-pyrrolo[2,3-*b*]pyridin-4-yl}acrylate [(XXI), (R9= TBDMS, n=0, R8= Ethyl)]**

537 mg (1.5 mmol) of 1-[*tert*-butyl(dimethyl)silyl]-4-iodo-1*H*-pyrrolo[2,3-*b*]pyridine (XXVIII) were dissolved in 5 mL of acetonitrile under argon atmosphere and 17 mg (0.075 mmol) of palladium acetate, 45 mg (0.15 mmol) of tris(o-tolyl)phosphine, 625 μL (4.5 mmol) of triethylamine and 0.5 mL (4.5 mmol) of ethylacrylate were added consecutively. The reaction mixture was heated at 100°C under stirring for 24 hours. The solvent was then removed in vacuo and the residue was triturated with n-hexane and collected by filtration. The crude was chromatographed on a silica gel column (eluent: CH2C12-n-hexane 4/1) to afford the title compound (250 mg 50 % yield) as an yellowish oil.

**<u>Ste 3.</u> ethyl (2*E*)-3-(1*H*-pyrrolo[2,3-*b*]pyridin-4-yl)acrylate [(XXII), (R9= TBDMS, n=0, R8 = Ethyl)]**

110 mg (0.33 mmol) of (2*E*)-3-{1-[*tert*-butyl(dimethyl)silyl]-1*H*-pyrrolo[2,3-*b*]pyridin-4-yl}acrylate were dissolved in 30 mL of ethanol 95% .206 mg (0.66 mmol) of bismuth trichloride, and 244 mg (6.6 mmol) of sodium borohydride were added at 0°C to the mixture portionwise. The reaction was stirred for 3 hours at the same temperature. 30 mL of water were then added and the suspension filtered through a celite pad. The filtrate was acidified with 1 N HCl, extracted with dichloromethane, dried over sodium sulfate and evaporated to dryness, to afford the title compound (100 mg 91 % yield) as an off-white solid.

**<u>Step 4.</u> ethyl 3-(1*H*-pyrrolo[2,3-*b*]pyridin-4-yl)propanoate [(XXIII), (n=0, R8 = Ethyl)]**

80 mg (0.24 mmol) of ethyl (2*E*)-3-{1-[*tert*-butyl(dimethyl)silyl]-1*H*-pyrrolo[2,3-*b*]pyridin-4-yl}propanoate were dissolved in 1 mL of tetrahydrofuran and 0.3 mL of 1 M tetrabutylammonium fluoride in THF were added under argon atmosphere. After 30 minutes under stirring at room temperature the mixture was poured into a saturated solution of sodium carbonate and extracted with dichloromethane. The organic layer was washed with water, dried over sodium sulfate and evaporated to dryness, to give the title compound (51 mg 100 % yield) as an pale yellow solid.
1H NMR (400 MHz, DMSO-$d_6$) δ ppm 11.98 (bs, 1H), 8.24 (d, *J*=5.95 Hz, 1H); 7.56 (d, *J*=3.52Hz, 1H); 7.22 (d, *J*=5.95 Hz, 1H); 6.75 (d, *J*=3.52Hz, 1H); 4.13 (q, *J*=7.33Hz, 2H); 3.38 (t, *J*=7.62Hz, 2H); 2.81 (t, *J*=7.62Hz, 2H); 1.22 (t, *J*=7.33Hz, 3H).

**<u>Step 5.</u> ethyl 3-(3-formyl-1*H*-pyrrolo[2,3-*b*]pyridin-4-yl)propanoate [(XXIV), (n=0, R8 = Ethyl)]**

90 mg (0.41 mmol) of ethyl 3-(1*H*-pyrrolo[2,3-*b*]pyridin-4-yl)propanoate were dissolved in 10 mL of 30 % acetic acid and 90 mg (0.64 mmol) of hexamethylentetramine were added. The reaction was heated at 100°C under stirring for 3 hours. After that time the mixture was cooled to room temperature and extracted several times with ethylacetate, washed with brine , dried over sodium sulfate and evaporated. The resulting crude was chromatographed on a silica gel column (eluent: CH2C12-CH3COCH3 4/1) to afford (40 mg 40 % yield) as an off-white solid.
1H NMR (400 MHz, DMSO-$d_6$) δ ppm 9.96 (s, 1H), 8.33 (m, 1H); 8.09 (s, 1H);4.13 (q, *J*=7.33Hz, 2H);3.67 (t, *J*=7.03Hz, 2H); 2.73 (t, *J*=7.03Hz, 2H); 1.22 (t, *J*=7.33Hz, 3H).

**<u>Step 6.</u> 3-(3-formyl-1*H*-pyrrolo[2,3-*b*]pyridin-4-yl)propanoic acid [(XXV), (n=0)]**

140 mg (0.57 mmol) of ethyl 3-(3-formyl-1*H*-pyrrolo[2,3-*b*]pyridin-4-yl)propanoate were suspended with 3 mL of ethanol and 1.5 mL of 1 N sodium hydrate were added at room temperature. After 3 hours under stirring the mixture was concentrated to small volume, the pH made neutral and the resulting precipitate collected by filtration, to obtain the title compound (100 mg 81 % yield) as an off-white solid.
1H NMR (400 MHz, DMSO-$d_6$) δ ppm 12.65 (bs, 1H), 9.85 (s, 1H), 8.40 (bs, 1H), 8.19 (d, *J*= 4.98 Hz, 1H), 7.10 (d, *J*= 4.98 Hz, 1H), 3.42 (t, *J*= 7.62 Hz, 2H), 2.36 (t, *J*= 7.62 Hz, 2H).

**<u>Step 7.</u> 1-acetyl-1,5-dihydropyrrolo[4,3,2-*ij*]isoquinoline-4-carboxylic acid [(XXVI), (n=1, R7= Methyl)]**

190 mg (0.87 mmol) of 3-(3-formyl-1*H*-pyrrolo[2,3-*b*]pyridin-4-yl)propanoic acid were suspended in 4 mL of acetic anhydride and 1.2 mL of triethylamine were added. The mixture was stirred at 100°C for 4 hours. After that time the reaction was cooled to room temperature and 20 mL of water were added the mixture stirred at 60°C for 10 minutes and then the solvent removed under reduced pressure. The residue was taken up several times with toluene and the

solvent evaporated again, to give, after trituration with diethylether, the title compound (80 mg 38 % yield) as a light tan solid.
1H NMR (400 MHz, DMSO-$d_6$) δ ppm 11.89 (bs, 1H)8.22 (s, 1H), 8.05 (s, 1H), 8.03 (d, *J*= 6.15 Hz, 1H), 7.28 (d, *J*= 6.15 Hz, 1H), 5.18 (s, 2H), 2.68 (s, 3H).

**Step 8. 1,2-dihydropyrrolo[4,3,2-*ij*]isoquinoline-4-carboxylic acid [(IXd), (A=-CH=, X= -CH=, Y= -$CH_2$-, R2=H)]**

80 mg (0.33 mmol) of 1-acetyl-1,5-dihydropyrrolo[4,3,2-*ij*]isoquinoline-4-carboxylic acid were dissolved in a mixture of 3 mL of water, 3 mL of 1N sodium hydrate and 10 mL of methanol. The solution was stirred at room temperature for 6 hours. The pH was made neutral with 1N hydrochloric acid and the solvent evaporated in vacuo. The residue was chromatographed on a silica gel column (eluent:CH2Cl2-CH3COOH-CH3OH 70/2/30, to afford the title compound (51 mg 77 % yield) as a off-white solid.
1H NMR (400 MHz, DMSO-$d_6$) δ ppm 11.88 (bs, 1H); 8.22 (s, 1H); 8.04 and 8.03 (2s, 2H); 7.28 (d, *J*=6.15 Hz, 1H); 5.18 (s, 2H); 2.68 (s, 3H);

**Step 9. *tert*-butyl {(2*S*)-2-[(1,2-dihydropyrrolo[4,3,2-*ij*]isoquinolin-4-ylcarbonyl)amino]-3-phenylpropyl}carbamate [(Id), (A= -CH=, X= -CH=, Y=-$CH_2$-, R2=H, R1 = (1*S*)-2-amino-1-benzylethyl)]**

20 mg (0.1 mmol) of 1,2-dihydropyrrolo[4,3,2-*ij*]isoquinoline-4-carboxylic acid were suspended in 5 mL of dry dichloromethane and 134 μL of DIPEA, 64 mg (0.1 mmol) of TBTU, 25 mg (0.1 mmol) of *tert*-butyl [(2*S*)-2-amino-3-phenylpropyl]carbamate were added consecutively to the mixture. The reaction was stirred at room temperature overnight. The solvent was then removed in vacuo, the residue redissolved with dichloromethane and washed with aqueous sodium hydrogenocarbonate. The organic phase was then dried over sodium sulfate and evaporated. The crude was purified by chromatography on a silica gel column (eluent: CH2Cl2-CH3OH 95/5) to afford the title compound (20 mg 46 % yield) as pale yellow solid.

**Step 10. *N*-[(1*S*)-2-amino-1-benzylethyl]-1,2-dihydropyrrolo[4,3,2-*ij*]isoquinoline-4-carboxamide dihydrochloride [(Id), (A= -CH=, X= -CH=, Y= -$CH_2$-, R2=H, R1 = (1*S*)-2-amino-1-benzylethyl)]**

20 mg (0.05 mmol) of *tert*-butyl {(2*S*)-2-[(1,2-dihydropyrrolo[4,3,2-*ij*]isoquinolin-4-ylcarbonyl)amino]-3-phenylpropyl}carbamate were dissolved in 3 mL of dry dioxane and 1 mL of 4 M HCl in dioxane were added. After 2 hours at room temperature under stirring the solvent was removed and the residue triturated with diethylether, and filtered. The title compound (18 mg 90 % yield) was obtained as a off white solid.

**Cpd. 7**

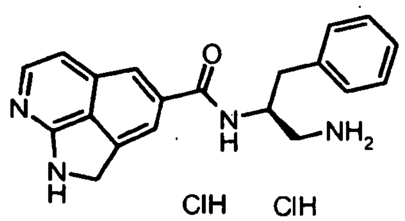

1H NMR (400 MHz, DMSO-$d_6$) δ ppm 8.84 (d, *J*=8.41 Hz, 1H), 8.13 (s, 1H); 8.06 (bs, 3H); 8.03 (s, 1H); 7.53 (d, *J*=7.19Hz, 1H); 7.03 (d, *J*=7.19Hz, 1H); 5.16 (s, 2H);4.49 (m, 1H);3.05 (t, *J*=5.61 Hz, 2H); 2.95 (m, 2H).
MS ESI (M+H) calc. 333.1710; found 333.1716 ($C_{20}H_{20}N_4O.2HCl$).
According to the same method the following compound can be prepared:

**N-(1S)-1-phenyl-2-pyrrolidin-1-ylethyl-1,2-dihydropyrrolo[4,3,2-ij]isoquinoline-4-carboxamide [(Id), (A= -CH=, X= -CH=, Y= -$CH_2$-, R2=H, R1= (1S)-1-phenyl-2-pyrrolidin-1-ylethyl)]**

**Cpd. 8**

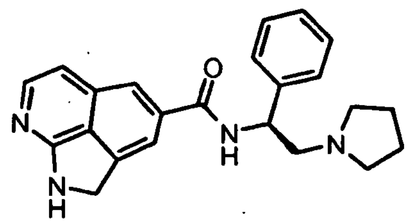

1H NMR (400 MHz, DMSO-$d_6$) δ ppm 8.91 (d, *J*=6.30 Hz, 1H), 7.93 (s, 2H); 7.78 (s, 1H);7.73 (d, *J*=6.30 Hz, 1H); 7.45 (m, 2H); 7.35 (m, 2H); 7.26 (m, I H); 6.74 (d, *J*=6.22 Hz, 1H); 5.24 (m, 1H); 4.82 (s, 1H);.
MS ESI (M+H) calc. 373.2023; found 373.2018 ($C_{23}H_{24}N_4O$).

## Claims

**1.** A compound of formula (I):

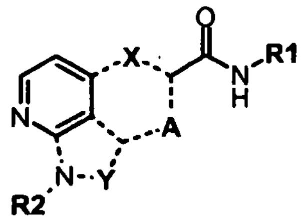

**(I)**

wherein

**R1** is hydrogen or an optionally substituted group selected from straight or branched $C_1$-$C_6$ alkyl, $C_3$-$C_6$ cycloalkyl, heterocycloalkyl, aryl, arylalkyl, heteroaryl and a group of formula (II):

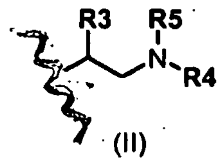

**(II)**

wherein the junction point between structure (II) and the -NH-function in formula (I) is the carbon atom bearing the R3 group;
**R2** is hydrogen or an optionally substituted group selected from straight or branched $C_1$-$C_6$ alkyl, aryalkyl and a group **-COR7;**
**R3** is an optionally substituted group selected from straight or branched $C_1$-$C_6$ alkyl, $C_3$-$C_6$ cycloalkyl, heterocycloalkyl, aryl, arylalkyl and heteroaryl;
**R4** and **R5** are hydrogen, or taken together with the nitrogen atom to which they are bonded, may form an optionally substituted heterocycloalkyl group;
**R7** is an optionally substituted group selected from straight or branched $C_1$-$C_6$ alkyl, $C_3$-$C_6$ cycloalkyl, hetero-

cycloalkyl, aryl, arylalkyl and heteroaryl;
**X** is -N(**R6**)-$CH_2$-, **A** is -$CH_2$-, **Y** is -CH= and **R6** is hydrogen or a group -CO**R7**, or
**X** is -N(**R6**)$CH_2$-, **A** is -CH=, **Y** is -CH= and **R6** is hydrogen or a group -CO**R7**, or
**X** is -$CH_2$-$CH_2$, **A** is -CH= and **Y** is -CH=, or
**X** is -CH=, **A** is -CH= and **Y** is -$CH_2$-, and
the dotted lines may represent a single, double or aromatic bond; or
or tautomers, complexes, solvates, hydrates or pharmaceutically acceptable salts thereof.

**2.** A compound according to claim 1 of formula (Ia):

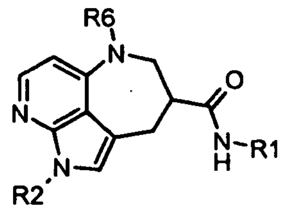

**(Ia)**

wherein:

**R1** and **R6** are as defined in claim 1,
**R2** is hydrogen or a group -CO**R7**;
**R7** is an optionally substituted straight or branched $C_1$-$C_6$ alkyl.

**3.** A compound of formula (Ib) according to claim 1,

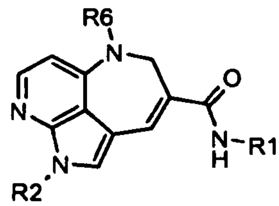

**(Ib)**

wherein:

**R1** and **R6** are as defined in claim 1,
**R2** is hydrogen or a group -CO**R7**;
**R7** is an optionally substituted straight or branched $C_1$-$C_6$ alkyl.

**4.** A compound of formula (Ic) according to claim 1,

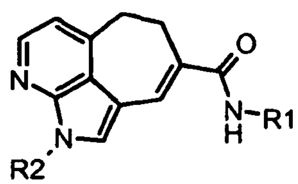

**(Ic)**

wherein:

**R1** is as defined in claim 1 and
**R2** is hydrogen or a group -CO**R7**;
**R7** is an optionally substituted straight or branched $C_1$-$C_6$ alkyl.

**5.** A compound of formula (Id) according to claim 1,

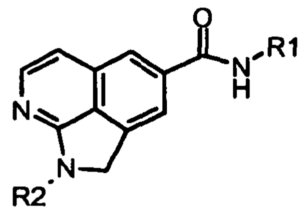

**(Id)**

wherein:

**R1** is as defined in claim 1,
**R2** is hydrogen or a group -CO**R7**;
**R7** is an optionally substituted straight or branched $C_1$-$C_6$ alkyl.

**6.** A compound or a pharmaceutically acceptable salt thereof according to claim 1, which is selected from the group consisting of:

*N*-[(1*S*)-2-amino-1-benzylethyl]-6,7-dihydro-2*H*-2,3-diazabenzo[*cd*]azutene-8-carboxamide;
*N*-[(1*S*)-2-amino-1-benzylethyl]-6,7-dihydro-2*H*-2,3,6-triazabenzo[*cd*]azulene-8-carboxamide;
6-acetyl-*N*-[(1*S*)-2-amino-1-benzylethyl]-6,7-dihydro-2*H*-2,3,6-triazabenzo[*cd*]azulene-8-carboxamide;
*N*-[(1*S*)-2-amino-1-benzylethyl]-6,7,8,9-tetrahydro-2*H*-2,3,6-triazabenzo[*cd*]azulene-8-carboxamide;
6,7,8,9-tetrahydro-2*H*-2,3,6-triazabenzo[*cd*]azulene-8-carboxamide;
*N*-[(1*S*)-1-phenyl-2-pyrrolidin-1-ylethyl]-6,7-dihydro-2*H*-2,3-diazabenzo[*cd*]azulene-8-carboxamide;
*N*-[(1*S*)-2-amino-1-benzylethyl]-1,2-dihydropyrrolo[4,3,2-*ij*]isoquinoline-4-carboxamide;
*N*-[(1*S*)-1-phenyl-2-pyrrolidin-1-ylethyl]-1,2-dihydropyrrolo[4,3,2-*ij*]isoquinoline-4-carboxamide;
6,7-dihydro-2H-2,3-diazabenzo[cd]azulene-8-carboxamide;
N-methyl-6,7-dihydro-2H-2,3-diazabenzo[cd]azulene-8-carboxamide;
N-benzyl-6,7-dihydro-2H-2,3-diazabenzo[cd]azulene-8-carboxamide;
N-(3-chlorobenzyl)-6,7-dihydro-2H-2,3-diazabenzo[cd]azulene-8-carboxamide;
N-(3-methoxybenzyl)-6,7-dihydro-2H-2,3-diazabenzo[cd]azulene-8-carboxamide;
N-[2-(dimethylamino)ethyl]-6,7-dihydro-2H-2,3-diazabenzo[cd]azulene-8-carboxamide;
N-(2-morpholin-4-ylethyl)-6,7-dihydro-2H-2,3-diazabenzo[cd]azulene-8-carboxamide;
N-[(1S)-2-morpholin-4-yl-1-phenylethyl]-6,7-dihydro-2H-2,3-diazabenzo[cd]azulene-8-carboxamide;
N-[(1S)-2-amino-1-phenylethyl]-6,7-dihydro-2H-2,3-diazabenzo[cd]azulene-8-carboxamide;
N-[(1S)-2-(4-methylpiperazin-1-yl)-1-phenylethyl]-6,7-dihydro-2H-2,3-diazabenzo[cd]azulene-8-carboxamide;
N-[(1S)-2-amino-1-(1H-indol-3-ylmethyl)ethyl]-6,7-dihydro-2H-2,3-diazabenzo[cd]azulene-8-carboxamide;
N-[(1S)-2-amino-1-(naphthalen-1-ylmethyl)ethyl]-6,7-dihydro-2H-2,3-diazabenzo[cd]azulene-8-carboxamide;
6,7-dihydro-2H-2,3,6-triazabenzo[cd]azulene-8-carboxamide;
N-methyl-6,7-dihydro-2H-2,3,6-triazabenzo[cd]azulene-8-carboxamide;
N-benzyl-6,7-dihydro-2H-2,3,6-triazabenzo[cd]azulene-8-carboxamide;
N-(3-chlorobenzyl)-6,7-dihydro-2H-2,3,6-triazabenzo[cd]azulene-8-carboxamide;
N-(3-methoxybenzyl)-6,7-dihydro-2H-2,3,6-triazabenzo[cd]azulene-8-carboxamide;
N-[2-(dimethylamino)ethyl]-6,7-dihydro-2H-2,3,6-triazabenzo[cd]azulene-8-carboxamide;
N-(2-morpholin-4-ylethyl)-6,7-dihydro-2H-2,3,6-triazabenzo[cd]azulene-8-carboxamide;
N-[(1S)-2-morpholin-4-yl-1-phenylethyl]-6,7-dihydro-2H-2,3,6-triazabenzo[cd]azulene-8-carboxamide;
N-[(1S)-2-amino-1-phenylethyl]-6,7-dihydro-2H-2,3,6-triazabenzo[cd]azulene-8-carboxamide;
N-[(1S)-2-(4-methylpiperazin-1-yl)-1-phenylethyl]-6,7-dihydro-2H-2,3,6-triazabenzo[cd]azulene-8-carboxamide;
N-[(1S)-2-amino-1-(1H-indol-3-ylmethyl)ethyl]-6,7-dihydro-2H-2,3,6-triazabenzo[cd]azulene-8-carboxamide;

N-[(1S)-2-ammo-1-(naphthaten-1-ylmethyl)ethyl]-6,7-dihydro-2H-2,3,6-triazabenzo[cd]azulene-8-carboxamide;
N-[(1S)-1-phenyl-2-pyrrolidin-1-ylethyl]-6,7-dihydro-2H-2,3,6-triazabenzo[cd]azulene-8-carboxamide;
6-acetyl-6,7-dihydro-2H-2,3,6-triazabenzo[cd]azulene-8-carboxamide;
6-acetyl-N-methyl-6,7-dihydro-2H-2,3,6-triazabenzo[cd]azulene-8-carboxamide;
6-acetyl-N-benzyl-6,7-dihydro-2H-2,3,6-triazabenzo[cd]azulene-8-carboxamide;
6-acetyl-N-(3-chlorobenzyl)-6,7-dihydro-2H-2,3,6-triazabenzo[cd]azulene-8-carboxamide;
6-acetyl-N-(3-methoxybenzyl)-6,7-dihydro-2H-2,3,6-triazabenzo[cd]azulene-8-carboxamide;
6-acetyl-N-[2-(dimethylamino)ethyl]-6,7-dihydro-2H-2,3,6-triazabenzo[cd]azulene-8-carboxamide;
6-acetyl-N-(2-morpholin-4-ylethyl)-6,7-dihydro-2H-2,3,6-triazabenzo[cd]azulene-8-carboxamide;
6-acetyl-N-[(1S)-2-morpholin-4-yl-1-phenylethyl]-6,7-dihydro-2H-2,3,6-triazabenzo[cd]azulene-8-carboxamide;
6-acetyl-N-[(1S)-2-amino-1-phenylethyl]-6,7-dihydro-2H-2,3,6-triazabenzo[cd]azulene-8-carboxamide;
6-acetyl-N-[(1S)-1-phenyl-2-pyrrolidin-1-ylethyl]-6,7-dihydro-2H-2,3,6-triazabenzo[cd]azulene-8-carboxamide;
6-acetyl-N-[(1S)-2-(4-methylpiperazin-1-yl)-1-phenylethyl]-6,7-dihydro-2H-2,3,6-triazabenzo[cd]azulene-8-carboxamide;
6-acetyl-N-[(S)-2-amino-1-(1H-indol-3-ylmethyl)ethyl]-6,7-dihydro-2H-2,3,6-triazabenzo[cd]azulene-8-carboxamide;
6-acetyl-N-[(1S)-2-amino-1-(naphthalen-1-ylmethyl)ethyl]-6,7-dihydro-2H-2,3,6-triazabenzo[cd]azulene-8-carboxamide;
1,2-dihydropyrrolo[4,3,2-ij]isoquinoline-4-carboxamide;
N-methyl-1,2-dihydropyrrolo[4,3,2-ij]isoquinoline-4-carboxamide;
N-benzyl-1,2-dihydropyrrolo[4,3,2-ij]isoquinoline-4-carboxamide;
N-(3-chlorobenzyl)-1,2-dihydropyrrolo[4,3,2-ij]isoquinoline-4-carboxamide;
N-(3-methoxybenzyl)-1,2-dihydropyrrolo[4,3,2-ij] isoquinoline-4-carboxamide;
N-[2-(dimethylamino)ethyl]-1,2-dihydropyrrolo[4,3,2-ij]isoquinoline-4-carboxamide; N-(2-morpholin-4-ylethyl)-1,2-dihydropyrrolo[4,3,2-ij]isoquinoline-4-carboxamide;
N-[(1S)-2-morpholin-4-yl-1-phenylethyl]-1,2-dihydropyrrolo[4,3,2-ij]isoquinoline-4-carboxamide;
N-[(1S)-2-amino-1-phenylethyl]-1,2-dihydropyrrolo [4,3,2-ij] isoquino line-4-carboxamide;
N-[(1S)-2-(4-methylpiperazin-1-yl)-1-phenylethyl]-1,2-dihydropyrrolo[4,3,2-ij] isoquino line-4-carboxamide;
N-[(1S)-2-amino-1-(1H-indol-3-ylmethyl)ethyl]-1,2-dihydropyrrolo[4,3,2-ij]isoquinoline-4-carboxamide and
N-[(1S)-2-amino-1-(naphthalen-1-ylmethyl)ethyl]-1,2-dihydropyrrolo[4,3,2-ij] isoquino line-4-carboxamide.

**7.** A process for preparing a compound of formula (I) as defined in claim 1, **characterized in that** the process comprises:

g) reacting a compound of formula (IXa), (IXb), (IXc) or (IXd):

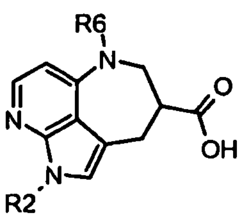
(IXa) OR
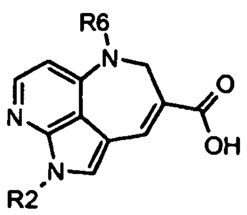
(IXb) OR
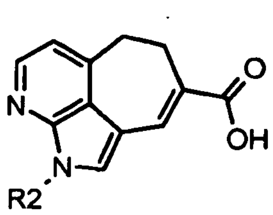
(IXc) OR
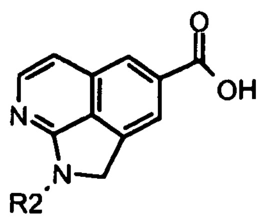
(IXd)

wherein **R2** and **R6** are as defined in claim 1, with an amine compound of formula (XIX):

**R1**-NH2 (XIX)

wherein **R1** is as defined in claim 1, to give a compound of formula (I), optionally separating the resulting compound into the single isomers, converting the compound of formula (I) into a different compound of formula (I) and/or into a pharmaceutically acceptable salt if desired, deprotecting the compound of formula (I) if necessary.

**8.** An in-vitro method for inhibiting the activity of one or more isoforms of the serin/threonine kinase AKT protein which comprises contacting the said protein with an effective amount of a compound as defined in claim 1.

**9.** A pharmaceutical composition comprising a therapeutically effective amount of a compound of formula (I) or a pharmaceutically acceptable salt thereof, as defined in claim 1, and at least one pharmaceutically acceptable excipient, carrier and/or diluent.

**10.** A pharmaceutical composition according to claim 9 further comprising one or more chemotherapeutic agents.

**11.** A product or kit comprising a compound of formula (I) or a pharmaceutically acceptable salt thereof, as defined in claim 1, or pharmaceutical compositions thereof as defined in claim 9 and one or more chemotherapeutic agents, as a combined preparation for simultaneous, separate or sequential use in anticancer therapy.

**12.** A compound of formula (I) or a pharmaceutically acceptable salt thereof, as defined in claim 1, for use as a medicament.

**13.** Use of a compound of formula (I) or a pharmaceutically acceptable salt thereof, as defined in claim 1, in the manufacture of a medicament with antitumor activity.

**14.** A compound of formula (I) or a pharmaceutically acceptable salt thereof, as defined in claim 1, for use in a method of treating cancer.

**Patentansprüche**

**1.** Verbindung der Formel (I):

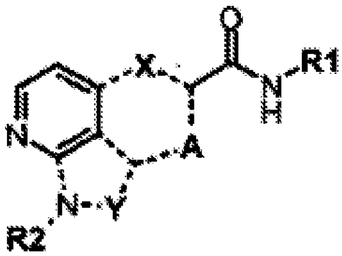

(I)

wobei R1 für Wasserstoff oder eine gegebenenfalls substituierte Gruppe steht, die aus linearen oder verzweigten $C_{1-6}$-Alkyl-, $C_{3-6}$-Cycloalkyl-, Heterocycloalkyl-, Aryl-, Arylalkyl-, Heteroarylgruppen und einer Gruppe der Formel (II) ausgewählt ist:

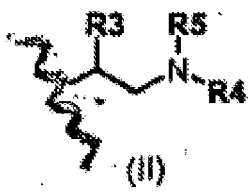

(II)

wobei der Verbindungspunkt zwischen der Struktur (II) und der NH-Gruppe der Formel (I) das Kohlenstoffatom ist, welches die R3-Gruppe trägt;
R2 für Wasserstoff oder eine gegebenenfalls substituierte Gruppe steht, die aus linearen oder verzweigten $C_{1-6}$-Alkylgruppen, Arylalkylgruppen und einer COR7-Gruppe ausgewählt ist;
R3 für Wasserstoff oder eine gegebenenfalls substituierte Gruppe steht, die aus linearen oder verzweigten $C_{1-6}$-Alkyl-, $C_{3-6}$-Cycloalkyl-, Heterocycloalkyl-, Aryl-, Arylalkyl- und Heteroarylgruppen ausgewählt ist;
R4 und R5 für Wasserstoff stehen oder zusammen mit dem Stickstoffatom, an welches sie gebunden sind, eine

gegebenenfalls substituierte Heterocycloalkylgruppe bilden können;
R7 für eine gegebenenfalls substituierte Gruppe steht, die aus linearen oder verzweigten $C_{1-6}$-Alkyl-, $C_{3-6}$-Cycloalkyl-, Heterocycloalkyl-, Aryl-, Arylalkyl- und Heteroarylgruppen ausgewählt ist;
X für -N (R6)-$CH_2$-, A für -$CH_2$-, Y für -CH= und R6 für Wasserstoff oder eine COR7-Gruppe steht oder
X für -N(R6)-$CH_2$-, A für -CH=, Y für -CH= und R6 für Wasserstoff oder eine COR7-Gruppe steht oder
X für -$CH_2$-$CH_2$-, A für -CH= und Y für -CH= steht oder
X für -CH=, A für -CH= und Y für -$CH_2$- steht und
die unterbrochenen Linien für eine Einfachbindung, eine Doppelbindung oder eine aromatische Bindung stehen können;

oder Tautomere, Komplexe, Solvate, Hydrate oder pharmazeutisch unbedenkliche Salze derselben.

**2.** Verbindung nach Anspruch 1 der Formel (Ia):

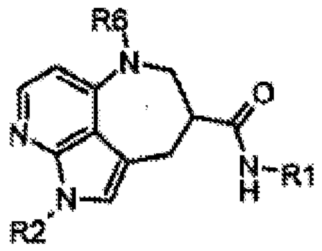

(Ia)

wobei:

R1 und R6 wie im Anspruch 1 definiert sind;
R2 für Wasserstoff oder eine COR7-Gruppe steht;
R7 für eine gegebenenfalls substituierte lineare oder verzweigte $C_{1-6}$-Alkylgruppe steht.

**3.** Verbindung nach Anspruch 1 der Formel (Ib):

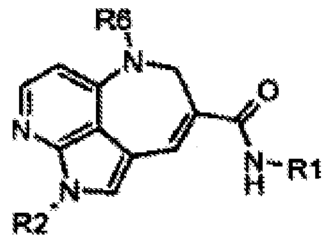

(Ib)

wobei:

R1 und R6 wie im Anspruch 1 definiert sind;
R2 für Wasserstoff oder eine COR7-Gruppe steht;
R7 für eine gegebenenfalls substituierte lineare oder verzweigte $C_{1-6}$-Alkylgruppe steht.

**4.** Verbindung nach Anspruch 1 der Formel (Ic):

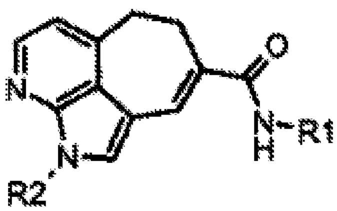

(Ic)

wobei:

R1 wie im Anspruch 1 definiert ist;
R2 für Wasserstoff oder eine COR7-Gruppe steht;
R7 für eine gegebenenfalls substituierte lineare oder verzweigte $C_{1-6}$-Alkylgruppe steht.

**5.** Verbindung nach Anspruch 1 der Formel (Id):

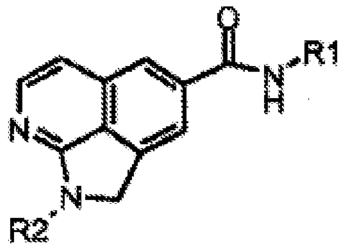

(Id)

wobei:

R1 wie im Anspruch 1 definiert ist;
R2 für Wasserstoff oder eine COR7-Gruppe steht;
R7 für eine gegebenenfalls substituierte lineare oder verzweigte $C_{1-6}$-Alkylgruppe steht.

**6.** Verbindung oder pharmazeutisch unbedenkliches Salz derselben nach Anspruch 1, welche(s) aus der Gruppe ausgewählt ist, die aus dem Folgenden besteht:

*N*-[(1S)-2-Amino-1-benzylethyl]-6,7-dihydro-2*H*-2,3-diazabenzo[*cd*]azulen-8-carboxamid;
*N*-[(1S)-2-Amino-1-benzylethyl]-6,7-dihydro-2*H*-2,3,6-triazabenzo[*cd*]azulen-8-carboxamid;
6-Acetyl-*N*-[(1S)-2-amino-1-benzylethyl]-6,7-dihydro-2*H*-2,3,6-triazabenzo[*cd*]azulen-8-carboxamid;
*N*-[(1S)-2-Amino-1-benzylethyl]-6,7,8,9-tetrahydro-2*H*-2,3,6-triazabenzo[*cd*]azulen-8-carboxamid;
6,7,8,9-Tetrahydro-2*H*-2,3,6-triazabenzo[*cd*]azulen-8-carboxamid;
*N*-[(1S)-1-Phenyl-2-pyrrolidin-1-ylethyl]-6,7-dihydro-2*H*-2,3-diazabenzo[*cd*]azulen-8-carboxamid;
*N*-[(1S)-2-Amino-1-benzylethyl]-1,2-dihydro-pyrrolo[4,3,2-*ij*]isochinolin-4-carboxamid;
*N*-[(1S)-1-Phenyl-2-pyrrolidin-1-ylethyl]-1,2-dihydro-pyrrolo[4,3,2-*ij*]isochinolin-4-carboxamid;
6,7-Dihydro-2H-2,3-diazabenzo[cd]azulen-8-carboxamid;
N-Methyl-6,7-dihydro-2H-2,3-diazabenzo[cd]azulen-8-carboxamid;
N-Benzyl-6,7-dihydro-2H-2,3-diazabenzo[cd]azulen-8-carboxamid;
N-(3-Chlorbenzyl)-6,7-dihydro-2H-2,3-diaza-benzo[cd]azulen-8-carboxamid;
N-(3-Methoxybenzyl)-6,7-dihydro-2H-2,3-diaza-benzo[cd]azulen-8-carboxamid;
N-[2-(Dimethylamino)ethyl]-6,7-dihydro-2H-2,3-diaza-benzo[cd]azulen-8-carboxamid;
N-(2-Morpholin-4-ylethyl)-6,7-dihydro-2H-2,3-diaza-benzo[cd]azulen-8-carboxamid;
N-[(1S)-2-Morpholin-4-yl-1-phenylethyl]-6,7-dihydro-2H-2,3-diazabenzo[cd]azulen-8-carboxamid;

N-[(1S)-2-Amino-1-phenylethyl]-6,7-dihydro-2H-2,3-diazabenzo[cd]azulen-8-carboxamid;
N-[(1S)-2-(4-Methylpiperazin-1-yl)-1-phenylethyl]-6,7-dihydro-2H-2,3-diazabenzo[cd]azulen-8-carboxamid;
N-[(1S)-2-Amino-1-(1H-indol-3-ylmethyl)ethyl]-6,7-dihydro-2H-2,3-diazabenzo[cd]azulen-8-carboxamid;
N-[(1S)-2-Amino-1-(naphthalin-1-ylmethyl)ethyl]-6,7-dihydro-2H-2,3-diazabenzo[cd]azulen-8-carboxamid;
6,7-Dihydro-2H-2,3,6-triazabenzo[cd]azulen-8-carboxamid;
N-Methyl-6,7-dihydro-2H-2,3,6-triazabenzo[cd]azulen-8-carboxamid;
N-Benzyl-6,7-dihydro-2H-2,3,6-triazabenzo[cd]azulen-8-carboxamid;
N-(3-Chlorbenzyl)-6,7-dihydro-2H-2,3,6-triazabenzo[cd]azulen-8-carboxamid;
N-(3-Methoxybenzyl)-6,7-dihydro-2H-2,3,6-triazabenzo[cd]azulen-8-carboxamid;
N-[2-(Dimethylamino)ethyl]-6,7-dihydro-2H-2,3,6-triazabenzo[cd]azulen-8-carboxamid;
N-(2-Morpholin-4-ylethyl)-6,7-dihydro-2H-2,3,6-triazabenzo[cd]azulen-8-carboxamid;
N-[(1S)-2-Morpholin-4-yl-1-phenylethyl]-6,7-dihydro-2H-2,3,6-triazabenzo[cd]azulen-8-carboxamid;
N-[(1S)-2-Amino-1-phenylethyl]-6,7-dihydro-2H-2,3,6-triazabenzo[cd]azulen-8-carboxamid;
N-[(1S)-2-(4-Methylpiperazin-1-yl)-1-phenylethyl]-6,7-dihydro-2H-2,3,6-triazabenzo[cd]azulen-8-carboxamid;
N-[(1S)-2-Amino-1-(1H-indol-3-ylmethyl)ethyl]-6,7-dihydro-2H-2,3,6-triazabenzo[cd]azulen-8-carboxamid;
N-[(1S)-2-Amino-1-(naphthalin-1-ylmethyl)ethyl]-6,7-dihydro-2H-2,3,6-triazabenzo[cd]azulen-8-carboxamid;
N-[(1S)-1-Phenyl-2-pyrrolidin-1-ylethyl]-6,7-dihydro-2H-2,3,6-triazabenzo[cd]azulen-8-carboxamid;
6-Acetyl-6,7-dihydro-2H-2,3,6-triazabenzo[cd]azulen-8-carboxamid;
6-Acetyl-N-methyl-6,7-dihydro-2H-2,3,6-triazabenzo[cd]azulen-8-carboxamid;
6-Acetyl-N-benzyl-6,7-dihydro-2H-2,3,6-triazabenzo[cd]azulen-8-carboxamid;
6-Acetyl-N-(3-chlorbenzyl)-6,7-dihydro-2H-2,3,6-triazabenzo[cd]azulen-8-carboxamid;
6-Acetyl-N-(3-methoxybenzyl)-6,7-dihydro-2H-2,3,6-triazabenzo[cd]azulen-8-carboxamid;
6-Acetyl-N-[2-(dimethylamino)ethyl]-6,7-dihydro-2H-2,3,6-triazabenzo[cd]azulen-8-carboxamid;
6-Acetyl-N-(2-morpholin-4-ylethyl)-6,7-dihydro-2H-2,3,6-triazabenzo[cd]azulen-8-carboxamid;
6-Acetyl-N-[(1S)-2-morpholin-4-yl-1-phenylethyl]-6,7-dihydro-2H-2,3,6-triazabenzo[cd]azulen-8-carboxamid;
6-Acetyl-N-[(1S)-2-amino-1-phenylethyl]-6,7-dihydro-2H-2,3,6-triazabenzo[cd]azulen-8-carboxamid;
6-Acetyl-N-[(1S)-1-phenyl-2-pyrrolidin-1-ylethyl]-6,7-dihydro-2H-2,3,6-triazabenzo[cd]azulen-8-carboxamid;
6-Acetyl-N-[(1S)-2-(4-methylpiperazin-1-yl)-1-phenylethyl]-6,7-dihydro-2H-2,3,6-triazabenzo[cd]azulen-8-carboxamid;
6-Acetyl-N-[(1S)-2-amino-1-(1H-indol-3-ylmethyl)ethyl]-6,7-dihydro-2H-2,3,6-triazabenzo[cd]azulen-8-carboxamid;
6-Acetyl-N-[(1S)-2-amino-1-(naphthalin-1-ylmethyl)ethyl]-6,7-dihydro-2H-2,3,6-triazabenzo[cd]azulen-8-carboxamid;
1,2-Dihydropyrrolo[4,3,2-*ij*]isochinolin-4-carboxamid;
N-Methyl-1,2-dihydropyrrolo[4,3,2-*ij*]isochinolin-4-carboxamid;
N-Benzyl-1,2-dihydropyrrolo[4,3,2-*ij*]isochinolin-4-carboxamid;
N-(3-Chlorbenzyl)-1,2-dihydropyrrolo[4,3,2-*ij*]isochinolin-4-carboxamid;
N-(3-Methoxybenzyl)-1,2-dihydropyrrolo[4,3,2-*ij*]isochinolin-4-carboxamid;
N-[2-(Dimethylamino)ethyl]-1,2-dihydropyrrolo[4,3,2-*ij*]isochinolin-4-carboxamid;
N-(2-Morpholin-4-ylethyl)-1,2-dihydropyrrolo[4,3,2-*ij*]isochinolin-4-carboxamid;
N-[(1S)-2-Morpholin-4-yl-1-phenylethyl]-1,2-dihydro-pyrrolo[4,3,2-*ij*]isochinolin-4-carboxamid;
N-[(1S)-2-Amino-1-phenylethyl]-1,2-dihydro-pyrrolo[4,3,2-*ij*]isochinolin-4-carboxamid;
N-[(1S)-2-(4-Methylpiperazin-1-yl)-1-phenylethyl]-1,2-dihydropyrrolo[4,3,2-*ij*]isochinolin-4-carboxamid;
N-[(1S)-2-Amino-1-(1H-indol-3-ylmethyl)ethyl]-1,2-dihydropyrrolo[4,3,2-*ij*]isochinolin-4-carboxamid;
N-[(1S)-2-Amino-1-(naphthalin-1-ylmethyl)ethyl]-1,2-dihydropyrrolo[4,3,2-*ij*]isochinolin-4-carboxamid.

**7.** Verfahren zur Herstellung einer Verbindung der Formel (I) nach Anspruch 1, **dadurch gekennzeichnet, dass** das Verfahren das Folgende umfasst:

Zur-Reaktion-Bringen einer Verbindung der Formel (IXa) , (IXb) , (IXc) oder (IXd) :

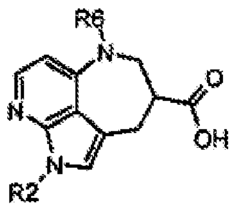
(IXa) ODER
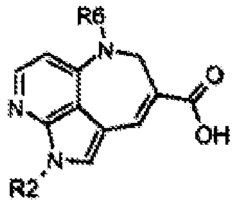
(IXb) ODER
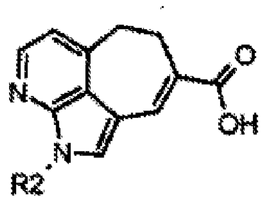
(IXc) ODER
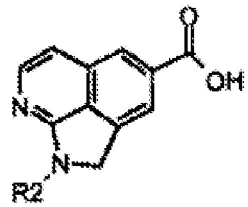
(IXd)

wobei R2 und R6 wie im Anspruch 1 definiert sind, mit einer Aminverbindung der Formel (XIX):

$R1\text{-}NH_2$ (XIX)

wobei R1 wie im Anspruch 1 definiert ist, wodurch man eine Verbindung der Formel (I) erhält, gegebenenfalls Trennen der resultierenden Verbindung in die einzelnen Isomere, Umformen der Verbindung der Formel (I) in eine andere Verbindung der Formel (I) und/oder in ein pharmazeutisch unbedenkliches Salz derselben, falls erwünscht, und Entfernen der Schutzgruppen der Verbindung der Formel (I), falls erforderlich.

**8.** In-vitro-Verfahren zum Hemmen der Aktivität einer oder mehrerer Isoformen des Serin-/Threoninkinase-AKT-Proteins, welches das In-Kontakt-Bringen des Proteins mit einer wirksamen Menge einer Verbindung nach Anspruch 1 umfasst.

**9.** Pharmazeutische Zusammensetzung, welche eine therapeutisch wirksame Menge einer Verbindung der Formel (I) oder eines pharmazeutisch unbedenklichen Salzes derselben nach Anspruch 1 und mindestens einen pharmazeutisch unbedenklichen Füllstoff, eine pharmazeutisch unbedenkliche Trägersubstanz und/oder ein pharmazeutisch unbedenkliches Verdünnungsmittel umfasst.

**10.** Pharmazeutische Zusammensetzung nach Anspruch 9, welche ferner ein oder mehrere Chemotherapeutika umfasst.

**11.** Produkt oder Zusammenstellung, welche(s) eine Verbindung der Formel (I) oder ein pharmazeutisch unbedenkliches Salz derselben nach Anspruch 1 oder pharmazeutische Zusammenstellungen derselben nach Anspruch 9 und ein oder mehrere Chemotherapeutika als Kombinationspräparat zur gleichzeitigen, getrennten oder zeitlich versetzten Anwendung in der Krebstherapie umfasst.

**12.** Verbindung der Formel (I) oder pharmazeutisch unbedenkliches Salz derselben nach Anspruch 1 zur Verwendung als Medikament.

**13.** Verwendung einer Verbindung der Formel (I) oder eines pharmazeutisch unbedenklichen Salzes derselben nach Anspruch 1 bei der Herstellung eines Medikaments mit Antitumoraktivität.

**14.** Verbindung der Formel (I) oder pharmazeutisch unbedenkliches Salz derselben nach Anspruch 1 zur Verwendung in einem Verfahren zur Behandlung von Krebs.

## Revendications

**1.** Composé de formule (I) :

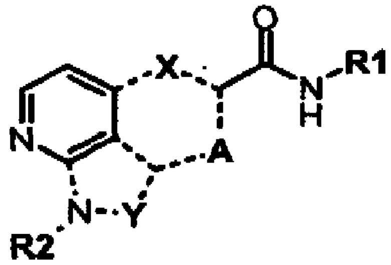

(I)

dans laquelle
R1 est de l'hydrogène ou un groupe facultativement substitué choisi parmi l'alkyle en $C_1$-$C_6$ linéaire ou ramifié, le cycloalkyle en $C_3$-$C_6$, l'hétérocycloalkyle, l'aryle, l'arylalkyle, l'hétéroaryle et un groupe de formule (II) :

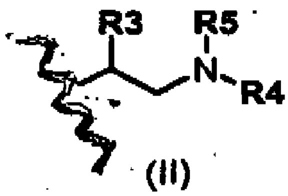

(II)

dans laquelle le point de jonction entre la structure (II) et la fonction -NH dans la formule (I) est l'atome de carbone portant le groupe R3 ;
R2 est de l'hydrogène ou un groupe facultativement substitué choisi parmi l'alkyle en $C_1$-$C_6$ linéaire ou ramifié, l'arylalkyle et un groupe -COR7 ;
R3 est un groupe facultativement substitué choisi parmi l'alkyle en $C_1$-$C_6$ linéaire ou ramifié, le cycloalkyle en $C_3$-$C_6$, l'hétérocycloalkyle, l'aryle, l'arylalkyle et l'hétéroaryle ;
R4 et R5 sont de l'hydrogène, ou pris conjointement avec l'atome d'azote auquel ils sont liés, peuvent former un groupe hétérocycloalkyle facultativement substitué ;
R7 est un groupe facultativement substitué choisi parmi l'alkyle en $C_1$-$C_6$ linéaire ou ramifié, le cycloalkyle en $C_3$-$C_6$, l'hétérocycloalkyle, l'aryle, l'arylalkyle et l'hétéroaryle ;
X est -N (R6) -$CH_2$-, A est -$CH_2$-, Y est -CH= et R6 est de l'hydrogène ou un groupe -COR7, ou
X est -N(R6)$CH_2$-, A est -CH=, Y est -CH= et R6 est de l'hydrogène ou un groupe -COR7, ou
X est -$CH_2$-$CH_2$, A est -CH= et Y est -CH=, ou
X est -CH=, A est -CH= et Y est -$CH_2$, et
les tirets peuvent représenter une liaison simple, double ou aromatique ; ou
ou les tautomères, complexes, solvantes, hydrates ou sels pharmaceutiquement acceptables de celui-ci.

**2.** Composé selon la revendication 1 de formule (Ia) :

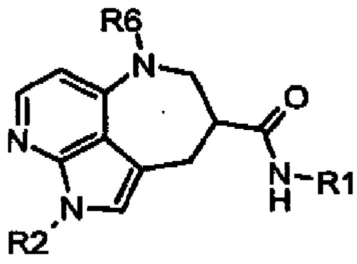

(Ia)

dans laquelle
R1 et R6 sont tels que définis dans la revendication 1,
R2 est de l'hydrogène ou un groupe -COR7 ;
R7 est un alkyle en $C_1$-$C_6$ linéaire ou ramifié facultativement substitué.

**3.** Composé selon la revendication 1 de formule (Ib) :

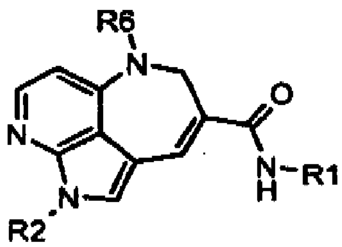

(Ib)

dans laquelle
R1 et R6 sont tels que définis dans la revendication 1,
R2 est de l'hydrogène ou un groupe -COR7 ;
R7 est un alkyle en $C_1$-$C_6$ linéaire ou ramifié facultativement substitué.

**4.** Composé selon la revendication 1 de formule (Ic) :

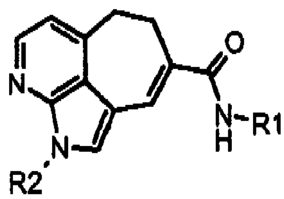

(Ic)

dans laquelle
R1 est tel que défini dans la revendication 1,
R2 est de l'hydrogène ou un groupe -COR7 ;
R7 est un alkyle en $C_1$-$C_6$ linéaire ou ramifié facultativement substitué.

**5.** Composé selon la revendication 1 de formule (Id) :

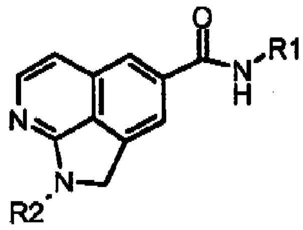

(Id)

dans laquelle
R1 est tel que défini dans la revendication 1,
R2 est de l'hydrogène ou un groupe -COR7 ;
R7 est un alkyle en $C_1$-$C_6$ linéaire ou ramifié facultativement substitué.

**6.** Composé ou sel pharmaceutiquement acceptable de celui-ci selon la revendication 1, choisi dans le groupe constitué par :

le *N*-[(1*S*)-2-amino-1-benzyléthyl]-6,7-dihydro-2*H*-2,3-diazabenzo[*cd*]azulène-8-carboxamide ;
le *N*-[(1*S*)-2-amino-1-benzyléthyl]-6,7-dihydro-2*H*-2,3,6-triazabenzo[*cd*]azulène-8-carboxamide ;
le 6-acétyl-*N*-[(1*S*)-2-amino-1-benzyléthyl]-6,7-dihydro-2*H*-2,3,6-triazabenzo[*cd*]azulène-8-carboxamide ;
le *N*-[(1*S*)-2-amino-1-benzyléthyl]-6,7,8,9-tétrahydro-2*H*-2,3,6-triazabenzo[*cd*]azulène-8-carboxamide ;
le 6,7,8,9-tétrahydro-2*H*-2,3,6-triazabenzo[*cd*]azulène-8-carboxamide ;
le *N*-[(1*S*)-1-phényl-2-pyrrolidin-1-yléthyl]-6,7-dihydro-2*H*-2,3-diazabenzo[*cd*]azulène-8-carboxamide ;
le *N*-[(1*S*)-2-amino-1-benzyléthyl]-1,2-dihydropyrrolo[4,3,2-*ij*]isoquinoléine-4-carboxamide ;
le *N*-[(1*S*)-1-phényl-2-pyrrolidin-1-yléthyl]-1,2-dihydropyrrolo[4,3,2-*ij*]isoquinoléine-4-carboxamide ;
le 6,7-dihydro-2*H*-2,3-diazabenzo[*cd*]azulène-8-carboxamide ;
le N-méthyl-6,7-dihydro-2*H*-2,3-diazabenzo[*cd*]azulène-8-carboxamide ;
le N-benzyl-6,7-dihydro-2*H*-2,3-diazabenzo[*cd*]azulène-8-carboxamide ;
le N-(3-chlorobenzyl)-6,7-dihydro-2*H*-2,3-diazabenzo[*cd*]azulène-8-carboxamide ;
le N-(3-méthoxybenzyl)-6,7-dihydro-2*H*-2,3-diazabenzo[*cd*]azulène-8-carboxamide ;
le N-[2-(diméthylamino)éthyl]-6,7-dihydro-2*H*-2,3-diazabenzo[*cd*]azulène-8-carboxamide ;
le N-(2-morpholin-4-yléthyl)-6,7-dihydro-2*H*-2,3-diazabenzo[*cd*]azulène-8-carboxamide ;
le N-[(1*S*)-2-morpholin-4-yl-1-phényléthyl]-6,7-dihydro-2*H*-2,3-diazabenzo[*cd*]azulène-8-carboxamide ;
le N-[(1*S*)-2-amino-1-phényléthyl]-6,7-dihydro-2*H*-2,3-diazabenzo[*cd*]azulène-8-carboxamide ;
le N-[(1*S*)-2-(4-méthylpipérazin-1-yl]-1-phényléthyl)-6,7-dihydro-2*H*-2,3-diazabenzo[*cd*]azulène-8-carboxamide ;
le N-[(1*S*)-2-amino-1-(1*H*-indol-3-ylméthyl)éthyl]-6,7-dihydro-2*H*-2,3-diazabenzo[*cd*]azulène-8-carboxamide ;
le N-[(1*S*)-2-amino-1-(naphtalèn-1-ylméthyl)éthyl]-6,7-dihydro-2*H*-2,3-diazabenzo[*cd*]azulène-8-carboxamide ;
le 6,7-dihydro-2*H*-2,3,6-triazabenzo[*cd*]azulène-8-carboxamide ;
le N-méthyl-6,7-dihydro-2*H*-2,3,6-triazabenzo[*cd*]azulène-8-carboxamide ;
le N-benzyl-6,7-dihydro-2*H*-2,3,6-triazabenzo[*cd*]azulène-8-carboxamide ;
le N-(3-chlorobenzyl)-6,7-dihydro-2*H*-2,3,6-triazabenzo[*cd*]azulène-8-carboxamide ;
le N-(3-méthoxybenzyl)-6,7-dihydro-2*H*-2,3,6-triazabenzo[*cd*]azulène-8-carboxamide ;
le N-[2-(diméthylamino)éthyl]-6,7-dihydro-2*H*-2,3,6-triazabenzo[*cd*]azulène-8-carboxamide ;
le N-(2-morpholin-4-yléthyl)-6,7-dihydro-2*H*-2,3,6-triazabenzo[*cd*]azulène-8-carboxamide ;
le N-[(1*S*)-2-morpholin-4-yl-1-phényléthyl]-6,7-dihydro-2*H*-2,3,6-triazabenzo[*cd*]azulène-8-carboxamide ;
le N-[(1*S*)-2-amino-1-phényléthyl]-6,7-dihydro-2*H*-2,3,6-triazabenzo[*cd*]azulène-8-carboxamide ;
le N-[(1*S*)-2-(4-méthylpipérazin-1-yl)-1-phényléthyl]-6,7-dihydro-2*H*-2,3,6-triazabenzo[*cd*]azulène-8-carboxamide ;
le N-[(1*S*)-2-amino-1-(1*H*-indol-3-ylméthyl)éthyl]-6,7-dihydro-2*H*-2,3,6-triazabenzo[*cd*]azulène-8-carboxamide ;
le N-[(1*S*)-2-amino-1-(naphtalèn-1-ylméthyl)éthyl]-6,7-dihydro-2*H*-2,3,6-triazabenzo[*cd*]azulène-8-carboxamide ;
le N-[(1*S*)-1-phényl-2-pyrrolidin-1-yléthyl]-6,7-dihydro-2*H*-2,3,6-triazabenzo[*cd*]azulène-8-carboxamide ;

le 6-acétyl-6,7-dihydro-2*H*-2,3,6-triazabenzo[*cd*]azulène-8-carboxamide ;
le 6-acétyl-N-méthyl-6,7-dihydro-2*H*-2,3,6-triazabenzo[*cd*]azulène-8-carboxamide ;
le 6-acétyl-N-benzyl-6,7-dihydro-2*H*-2,3,6-triazabenzo[*cd*]azulène-8-carboxamide ;
le 6-acétyl-N-(3-chlorobenzyl)-6,7-dihydro-2*H*-2,3,6-triazabenzo[*cd*]azulène-8-carboxamide ;
le 6-acétyl-N-(3-méthoxybenzyl)-6,7-dihydro-2*H*-2,3,6-triazabenzo[*cd*]azulène-8-carboxamide ;
le 6-acétyl-N-[2-(diméthylamino)éthyl]-6,7-dihydro-2*H*-2,3,6-triazabenzo[*cd*]azulène-8-carboxamide ;
le 6-acétyl-N-(2-morpholin-4-yléthyl]-6,7-dihydro-2*H*-2,3,6-triazabenzo[*cd*]azulène-8-carboxamide ;
le 6-acétyl-N-[(1S)-2-morpholin-4-yl-l-phényléthyl]-6,7-dihydro-2*H*-2,3,6-triazabenzo[*cd*]azulène-8-carboxamide ;
le 6-acétyl-N-[(1S)-2-amino-1-phényléthyl]-6,7-dihydro-2*H*-2,3,6-triazabenzo[*cd*]azulène-8-carboxamide ;
le 6-acétyl-N-[(1S)-1-phényl-2-pyrrolidin-1-yléthyl]-6,7-dihydro-2*H*-2,3,6-triazabenzo[*cd*]azulène-8-carboxamide ;
le 6-acétyl-N-[(1S)-2-(4-méthylpipérazin-1-yl)-1-phényléthyl]-6,7-dihydro-2*H*-2,3,6-triazabenzo[*cd*]azulène-8-carboxamide ;
le 6-acétyl-N-[(1S)-2-amino-1-(1*H*-indol-3-ylméthyl)éthyl]-6,7-dihydro-2*H*-2,3,6-triazabenzo[*cd*]azulène-8-carboxamide ;
le 6-acétyl-N-[(1S)-2-amino-1-(naphtalèn-1-ylméthyl)éthyl]-6,7-dihydro-2*H*-2,3,6-triazabenzo[*cd*]azulène-8-carboxamide ;
le 1,2-dihydropyrrolo[4,3,2-*ij*]isoquinoléine-4-carboxamide ;
le N-méthyl-1,2-dihydropyrrolo[4,3,2-*ij*]isoquinoléine-4-carboxamide ;
le N-benzyl-1,2-dihydropyrrolo[4,3,2-*ij*]isoquinoléine-4-carboxamide ;
le N-(3-chlorobenzyl)-1,2-dihydropyrrolo[4,3,2-*ij*]isoquinoléine-4-carboxamide ;
le N-(3-méthoxybenzyl)-1,2-dihydropyrrolo[4,3,2-*ij*]isoquinoléine-4-carboxamide ;
le N-[2-(diméthylamino)éthyl]-1,2-dihydropyrrolo[4,3,2-*ij*]isoquinoléine-4-carboxamide ;
le N-(2-morpholin-4-yléthyl)-1,2-dihydropyrrolo[4,3,2-*ij*]isoquinoléine-4-carboxamide ;
le N-[(1*S*)-2-morpholin-4-yl-1-phényléthyl]-1,2-dihydropyrrolo[4,3,2-*ij*]isoquinoléine-4-carboxamide ;
le N-[(1*S*)-2-amino-4-yl-1-phényléthyl]-1,2-dihydropyrrolo[4,3,2-*ij*]isoquinoléine-4-carboxamide ;
le N-[(1*S*)-2-(4-méthylpipérazin-1-yl)-1-phényléthyl]-1,2-dihydropyrrolo[4,3,2-*ij*]isoquinoléine-4-carboxamide ;
le N-[(1*S*)-2-amino-1-(1*H*-indol-3-ylméthyl)éthyl]-1,2-dihydropyrrolo[4,3,2-*ij*]isoquinoléine-4-carboxamide ; et
le N-[(1*S*)-2-amino-1-(naphtalèn-1-ylméthyl)éthyl]-1,2-dihydropyrrolo[4,3,2-*ij*]isoquinoléine-4-carboxamide.

**7.** Procédé de préparation d'un composé de formule (I) tel que défini dans la revendication 1, **caractérisé en ce que** le procédé comprend :

g) la mise en réaction d'un composé de formule (IXa) , (IXb) , (IXc) ou (IXd) :

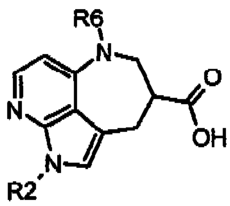
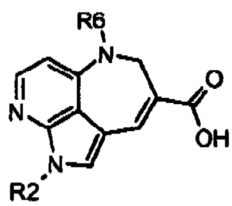
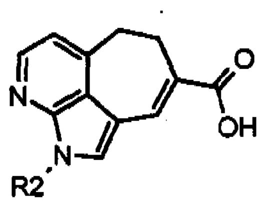
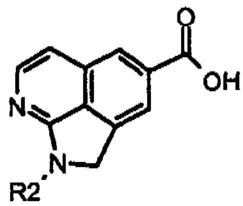

(IXa) OR (IXb) OR (IXc) OR (IXd)

dans laquelle R2 et R6 sont tels que définis dans la revendication 1, avec un composé amino de formule (XIX) :

**R1-NH2** **(XIX)**

dans laquelle R1 est tel que défini dans la revendication 1, pour donner un composé de formule (I), la séparation facultative du composé obtenu en isomères uniques, la conversion du composé de formule (I) en un composé de formule (I) différent et/ou en un sel pharmaceutiquement acceptable si souhaité, la déprotection du composé de formule (I) si nécessaire.

**8.** Méthode in vitro d'inhibition de l'activité d'une ou plusieurs isoformes de la protéine sérine/thréonine kinase AKT

qui comprend la mise en contact de ladite protéine avec une quantité efficace d'un composé tel que défini dans la revendication 1.

**9.** Composition pharmaceutique comprenant une quantité thérapeutiquement efficace d'un composé de formule (I) ou d'un sel pharmaceutiquement acceptable de celui-ci, tel que défini dans la revendication 1, et au moins un excipient, support et/ou diluant pharmaceutiquement acceptable.

**10.** Composition pharmaceutique selon la revendication 9, comprenant en outre un ou plusieurs agents chimiothérapeutiques.

**11.** Produit ou kit comprenant un composé de formule (I) ou un sel pharmaceutiquement acceptable de celui-ci, tel que défini dans la revendication 1, ou compositions pharmaceutiques de celui-ci telles que définies dans la revendication 9 et un ou plusieurs agents chimiothérapeutiques, sous forme de préparation combinée pour une utilisation simultanée, séparée ou séquentielle dans le cadre de la thérapie anticancéreuse.

**12.** Composé de formule (I) ou sel pharmaceutiquement acceptable de celui-ci, tel que défini dans la revendication 1, pour une utilisation comme médicament.

**13.** Utilisation d'un composé de formule (I) ou sel pharmaceutiquement acceptable de celui-ci, tel que défini dans la revendication 1, dans la fabrication d'un médicament ayant une activité antitumorale.

**14.** Composé de formule (I) ou sel pharmaceutiquement acceptable de celui-ci, tel que défini dans la revendication 1, pour une utilisation dans une méthode de traitement du cancer.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2003037862 A
- WO 200198299 A
- WO 2001002369 A2


### Non-patent literature cited in the description


- *Current Opinion in Chemical Biology,* 1999, vol. 3, 459-465
- *Proc. Natl. Acad. Sci.,* 2001, vol. 98, 10983-10985
- *J. Cell. Sci.,* 2001, vol. 114, 2903-2910
- *Circ. Res.,* 2000, vol. 86, 15-23
- *Proc. Natl. Acad. Sci.,* 1992, vol. 89, 9267-9271
- *J. Biol. Chem.,* 1999, vol. 274, 9133-9136
- *Cell. Signal.,* 2002, vol. 14, 381-395
- *Mol. Cell. Biol.,* 1997, vol. 17, 1595-1606
- *Science,* 1997, vol. 275, 628-630
- *Genev. Dev.,* 1999, vol. 13, 2905-2927
- *Current Biology,* 2002, vol. 12, 1251-1255
- *J. Biol. Chem.,* 2003, vol. 278, 21615-21622
- *Eur. J. Biochem.,* 1999, vol. 263, 605-611
- *Cancer Res.,* 1997, vol. 57, 2124-2129
- *Nat. Cell. Biol.,* 1999, vol. 1, 500-506
- *J. Biol. Chem.,* 1999, vol. 274, 1865-1868
- *J. Clin. Invest.,* 1999, vol. 106, 493-499
- *Nat. Med.,* 2000, vol. 6, 1004-1010
- *Proc. Natl. Acad. Sci,* 1996, vol. 93, 3636-3641
- *Int. J. Cancer,* 1995, vol. 64, 280-285
- *Nature,* 1999, vol. 401, 33-34
- *Oncogene,* 2000, vol. 19, 2324-2330
- *Neurosci.,* 2000, vol. 20, 2875-2886
- *Proc. Natl. Acad.,* 2001, vol. 98, 7200-7205
- *Nature,* 2001, vol. 411, 355-365
- *Nat. Rev. Cancer,* 2002, vol. 2, 489-501
- **Cohen, S.G. et al.** *J. Am. Chem. Soc.,* 1961, vol. 83, 4225-28
- **Green, Theodora W. ; Wuts, Peter G.M.** - Protective Groups in Organic Synthesis. John Wiley & Sons Inc, 1999
- **E.L.Eliel, S.H.Wilen ; L.N. Mander.** Stereochemistry of Organic Compounds. Wiley-Interscience, 1994
- **Kumar, C. C.** *Biochem. Biophys. ACTA,* 2001, vol. 1526, 257-268
- **Alessi, D. R.** *Biochem. J.,* 1998, vol. 331, 299-308
- **C. Dalvit et al.** *J. Am. Chem. Soc.,* 2003, vol. 125 (47), 14620